# FASCICULE DE BREVET EUROPEEN

(11) **EP 0 589 952 B1**
(45) Date de publication et mention de la délivrance du brevet: **27.08.1997**
(21) Numéro de dépôt: 92911884.2
(22) Date de dépôt: 10.06.1992
(51) Int. Cl.: C07C 323/36, C07C 323/37, C07C 323/41, C07C 323/43, C07C 323/44, A61K 7/13

(54) **UTILISATION DE METAAMINOPHENOLS SOUFRES POUR LA TEINTURE DES FIBRES KERATINIQUES, COMPOSITIONS TINCTORIALES ET COMPOSES NOUVEAUX MIS EN OEUVRE**
VERWENDUNG VON SCHWEFELHALTIGEN META-AMINOPHENOLEN ZUM FÄRBEN VON KERATINFASERN, FÄRBEZUSAMMENSETZUNGEN UND NEUE VERBINDUNGEN
USE OF SUPLHUR METAAMINOPHENOLS FOR DYEING KERATINIC FIBRES, TINCTORIAL COMPOSITIONS AND NOVEL COMPOUNDS

(30) Priorité: 13.06.1991 FR 9107248
(43) Date de publication de la demande: 06.04.1994
(73) Titulaire: L'OREAL, 75008 Paris (FR)
(72) Inventeur: LAGRANGE, Alain, F-78400 Chatou (FR); GENET, Alain, F-93600 Aulnay-sous-Bois (FR); JUNINO, Alex, F-93190 Livry-Gargan (FR)
(74) Mandataire: Casalonga, Axel
(86) Numéro de dépôt international: FR9200524
(87) Numéro de publication internationale: WO9222525

(56) Documents cités:
- EP-A- 0 331 144
- FR-A- 2 547 300
- GB-A- 718 322
- GB-A- 1 122 323
- US-A- 4 200 466
- CHEMISCHE BERICHTE, vol. 90, 1957, Weinheim, DE, pages 2471-2479; R. PFLEGER et al: "Über anomale quartäre Salze von O-Aminophenolen (Semiphenolbetaine)"
- JOURNAL OF CHROMATOGRAPHY, vol. 474, no. 1, 1989, Amsterdam, NL, pages 209-222; M. S. RASHED et al: "Use of thermospray liquid chromatography-mass spectrometry for characterisation of reactive metabolites of 3alpha-hydroxyacetanilide"
- PHOSPHORUS SULFUR, vol. 2, no. 1-3, 1976, London, GB, pages 133-139; T. S. CROFT: "Fluoroalkylthio substituted aromatic derivatives"

## Description

La présente invention est relative à l'utilisation de métaaminophénols soufrés pour la teinture des fibres kératiniques, en particulier des cheveux humains, aux compositions tinctoriales, aux procédés de teinture et à de nouveaux composés de la famille des métaaminophénols soufrés.

La Demanderesse a proposé dans des demandes antérieures l'utilisation de certains composés méta-aminophénol en teinture par oxydation de fibres kératiniques. Dans sa demande FR-A-2 547 300, la Demanderesse décrit l'utilisation de tels composés, substitués en outre en position para par rapport au groupement amino par un radical -OZ. Ces composés sont associés à des précurseurs de colorants d'oxydation pour l'obtention de teintes essentiellement dans les nuances rouge, cuivre, acajou, pourpre et aux reflets rosés, parmes, violacés.

Dans sa demande EP-A-331 144, la Demanderesse indique l'existence d'une équivalence dans le cadre de l'invention décrite, qui traite de l'utilisation de para-aminophénol en teinture oxydative, entre le groupement -OZ et le groupement -SZ.

Des métaaminophénols soufrés sont décrits dans le document GB-A-718 322 comme possédant des propriétés bactéricides et vermicides.

Les composés utilisés pour la teinture des fibres kératiniques, en particulier des cheveux humains, conformes à l'invention et qui confèrent des colorations différentes que celles obtenues dans la demande FR-A-2 547 300, répondent à la formule : 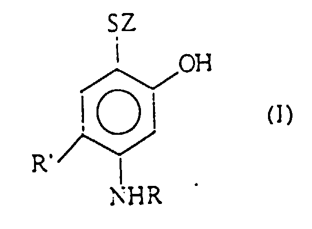 dans laquelle Z représente un radical alkyle en C₁-C₁₈, un radical aryle, aralkyle dans lequel le radical alkyle comporte 1 à 6 atomes de carbone, un radical monohydroxyalkyle contenant 1 à 6 atomes de carbone ou polyhydroxyalkyle en C₂-C₆, un radical aminoalkyle de formule : 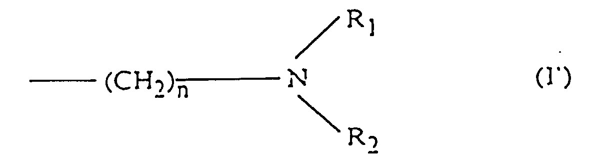 dans laquelle n est un nombre entier compris entre 1 et 6 inclus, R₁ et R₂, identiques ou différents, représentent un atome d'hydrogène ou un radical alkyle en C₁-C₄, hydroxyalkyle en C₁-C₄, acyle en C₂-C₆ ; R représente un atome d'hydrogène, un radical alkyle en C₁-C₆, un radical monohydroxyalkyle en C₁-C₆, polyhydroxyalkyle en C₂-C₆, monocarbamylalkyle en C₁-C₆, dicarbamylalkyle en C₁-C₆, aminoalkyle en C₁-C₆, acyle en C₁-C₆, carbalcoxy en C₂-C₆ , carbamyle ou monoalkyle en C₁-C₆ carbamyle, R' représente un atome d'hydrogène, un radical alkyle en C₁-C₄, thioalkyle en C₁-C₄ ou alcoxy en C₁-C₄, ainsi que les sels d'acide correspondants aux composés de formule (I).

Parmi les significations préférées du radical Z, on peut citer pour la signification alkyle en C₁₋C₁₈, les radicaux méthyle, éthyle, propyle, butyle ; le radical aralkyle désigne benzyle; le radical aryle désigne phényle, le radical mono- ou polyhydroxyalkyle désigne de préférence -CH₂-CH₂OH, -CH₂CHOH-CH₂-OH, -CH₂CHOH-CH₃, aminoalkyle désigne de préférence -CH₂-CH₂-NH₂, -CH₂-CH₂-NHCH₃, 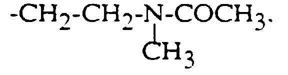 -CH₂-CH₂-NHCOCH₃; lorsque R représente acyle, il désigne de préférence formyle, acétyle et propionyle.

Les sels d'acide sont choisis de préférence parmi les chlorhydrates, sulfates ou bromhydrates.

Les composés particulièrement préférés entrant dans la définition de la formule (I), sont choisis parmi :
- le 2-méthylthio 5-aminophénol,
- le 2-éthylthio 5-aminophénol,
- le 2-butylthio 5-aminophénol,
- le 2-benzylthio 5-aminophénol,
- le 2-(β-hydroxyéthylthio) 5-aminophénol,
- le 2-(β-acétylaminoéthylthio) 5-aminophénol,
- le 2-méthylthio 5-uréidophénol,
- le 2-méthylthio 5-acétylaminophénol,
- le 2-méthylthio 5-carbéthoxyamino phénol,
- le 2,4-bis-méthylthio 5-aminophénol,
- le 2-(4'-amino 2'-hydroxyphényl)thio 5-aminophénol,
- le 2-(4'-nitrophényl)thio 5-aminophénol,
- le 2-(2'-aminophényl)thio 5-aminophénol.

Les composés répondant à la formule (I) sont plus particulièrement utilisés comme coupleurs en présence de précurseurs de colorant d'oxydation para ou ortho connus en eux-mêmes, permettant de teindre les fibres kératiniques et en particulier les cheveux, selon un processus mettant en oeuvre une réaction de condensation oxydative des précurseurs et du ou des coupleurs en présence.

Parmi les composés utilisés conformément à l'invention, il y a des composés nouveaux qui constituent un autre objet de l'invention. Ces composés répondent à la formule (IA) : 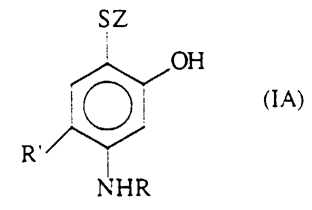 dans laquelle Z représente un radical alkyle en C₁-C₁₈, un radical aralkyle dans lequel le radical alkyle comporte 1 à 6 atomes de carbone, un radical monohydroxyalkyle en C₁-C₆, un radical polyhydroxyalkyle en C₂-C₆, un radical aminoalkyle de formule : 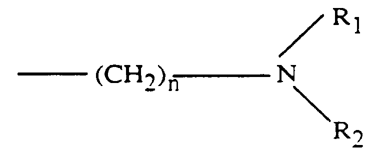 dans laquelle n est un nombre entier compris entre 1 et 6 inclus, R₁ et R₂, identiques ou différents, représentent un atome d'hydrogène ou un radical alkyle en C₁-C₄, hydroxyalkyle en C₁-C₄, acyle en C₂-C₆ ; R représente un atome d'hydrogène, un radical alkyle en C₁-C₆, un radical monohydroxyalkyle en C₁-C₆, polyhydroxyalkyle en C₂-C₆, monocarbamylalkyle en C₁-C₆, dicarbamylalkyle en C₁-C₆, aminoalkyle en C₁-C₆, acyle en C₁-C₆, carbalcoxy en C₂-C₆, carbamyle ou monoalkyle en C₁-C₆ carbamyle, R' représente un atome d'hydrogène, un radical alkyle en C₁-C₄, thioalkyle en C₁-C₄ ou alcoxy en C₁-C₄, sous réserve que lorsque R et R' désignent un atome d'hydrogène, Z ne peut désigner les radicaux butyle, β,γ-dihydroxypropyle et diéthylaminoéthyle, ainsi que les sels d'acide correspondants.

Les composés de formule (I) ou leurs sels peuvent être préparés selon un procédé consistant dans une première étape, à faire réagir en présence d'une base telle que la potasse, le carbonate de potassium, le 3,4-méthylènedioxy 1-nitrobenzène éventuellement substitué en 6 par un groupement alkyle, thioalkyle ou alcoxy sur un thiol de formule (II) :

Z-SH (II)

dans laquelle Z représente, soit un groupement alkyle en C₁-C₁₈, un groupement aralkyle dans lequel le radical alkyle est en C₁₋C₆, soit un groupement monohydroxyalkyle ayant 1 à 6 atomes de carbone polyhydroxyalkyle en C₂-C₆, un groupement de formule : 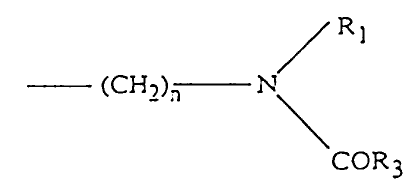 où R₁ et n ont les significations indiquées ci-dessus et où R₃ représente un atome d'hydrogène ou un radical alkyle ayant 1 à 3 atomes de carbone ; dans une deuxième étape, on réduit le groupement NO₂ du composé de formule (III) : 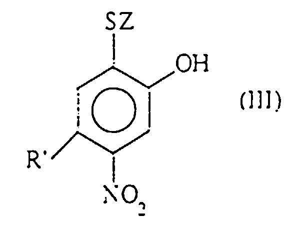 où Z a la même signification que dans la formule (I) et R' représente hydrogène, alkyle, alcoxy ou thioalkyle,
obtenu précédemment pour préparer un composé répondant à la formule (IV) : 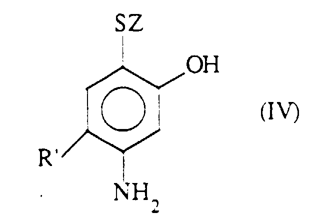 dans laquelle Z et R' ont a la signification indiquée ci-dessus ; éventuellement dans une troisième étape, et selon le composé de formule (I) que l'on désire, on transforme le composé de formule (IV), soit par hydrolyse acide, notamment chlorhydrique lorsque Z comprend un groupement amine acylé, soit par substitution de l'amine extra-nucléaire lorsque Z comporte un groupement amine, soit par mono-substitution de l'amine aromatique.

Dans le cas particulier où Z représente un alkyle et R' thioalkyle, les composés de formule (I) peuvent être préparés par réaction, en premier lieu, d'un excès de thioalkylate alcalin ZSM où Z = alkyle et M = métal alcalin, sur le 3,4-méthylènedioxy 6-trifluoroéthyle nitrobenzène, pour obtenir un composé de formule (IIIA) : 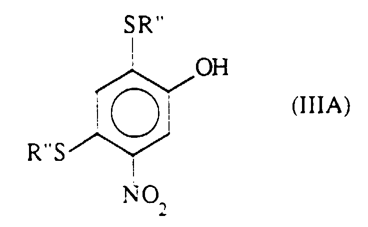 où R" = alkyle, qui, dans une deuxième étape, est soumis à une réduction.

La réduction du groupe nitro des composés de formules (III) et (IIIA) s'effectue de préférence en utilisant du fer en mileu acétique, ou alors par le cyclohexène en présence d'un catalyseur palladium-charbon ou par tout autre procédé de réduction classique.

Lorsque Z comporte un groupe amine acylé et que l'on soumet le composé de formule (IV) à une hydrolyse acide, on obtient un composé de formule (V) : 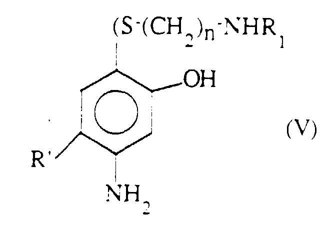 dans laquelle n, R₁ et R' ont les significations indiquées ci-dessus.

L'ensemble des composés de formule (IV) et (V) et de leurs dérivés obtenus à partir d'eux par substitution de l'amine aromatique ou extra-nucléaire, est couvert par les composés de formule (I).

Pour la substitution des amines aromatiques ou extra-nucléaires, on peut faire réagir, par exemple, le bromure d'éthyle, la bromhydrine du glycol, l'éthyl-chloroformiate, la β-chloracétamide, l'anhydride acétique.

L'utilisation pour la teinture des fibres kératiniques des composés de formule (I) s'effectue plus particulièrement à l'aide de compositions tinctoriales dites d'oxydation.

La composition tinctoriale qui constitue un autre objet de l'invention est essentiellement caractérisée par le fait qu'elle contient, dans un milieu approprié pour la teinture des fibres kératiniques, cosmétiquement acceptable, lorsque les fibres sont des cheveux, un précurseur de colorant d'oxydation de type para ou onho et au moins un métaaminophénol soufré de formule (I) défini ci-dessus.

Les précurseurs de colorants de types para ou ortho utilisés conformément à l'invention, sont des composés qui ne sont pas des colorants en eux-mêmes, mais qui forment des colorants par un processus de condensation oxydative, soit sur eux-mêmes, soit en présence d'un coupleur ou modificateur.

Ces précurseurs de colorants d'oxydation de type para ou ortho sont des composés benzéniques ou hétérocycliques qui comportent deux groupements amino ou bien un groupement amino et un groupement hydroxyle en position para ou ortho, l'un par rapport à l'autre.

Les précurseurs de colorants d'oxydation de type para ou ortho peuvent être choisis parmi les paraphénylènediamines, les paraaminophénols, les précurseurs hétérocycliques para dérivés de la pyridine ou de la pyrimidine, tels que la 2,5-diaminopyridine, la 2-hydroxy 5-aminopyridine, la 2,4,5,6-tétraaminopyrimidine, la 4,5-diamino 1-méthylpyrazole, la 2-diméthylamino 4,5,6-triaminopyrimidine, les orthoaminophénols et les bases dites "doubles".

A titre de paraphénylènediamines, on peut plus particulièrement citer les composés répondant à la formule (VI) : 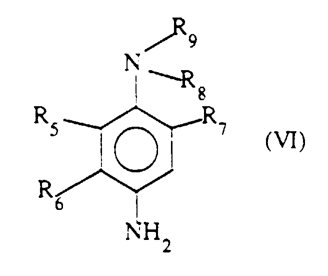 dans laquelle :
R₅, R₆, R₇, identiques ou différents, représentent un atome d'hydrogène ou d'halogène, un radical alkyle, un radical alcoxy, un radical carboxy, sulfo, hydroxyalkyle en C₁-C₄;
R₈ et R₉, identiques ou différents, représentent un atome d'hydrogène, un radidal alkyle, hydroxyalkyle, alcoxyalkyle, carbamylalkyle, mésylaminoalkyle, acétylaminoalkyle, uréidoalkyle, carbalcoxyaminoalkyle, sulfoalkyle, pipéridinoalkyle, morpholinoalkyle, phényle éventuellement substitué en para par un groupement amino ; ou bien R₈ et R₉ forment, conjointement avec l'atome d'azote auquel ils sont liés, un hétérocycle pipéridino ou morpholino, sous réserve que R₅ ou R₇ représentent un atome d'hydrogène lorsque R₈ et R₉ ne représentent pas hydrogène, ainsi que les sels de ses composés.

Les radicaux alkyle ou alcoxy désignent de préférence un groupement ayant 1 à 4 atomes de carbone et notamment méthyle, éthyle, propyle, méthoxy, éthoxy.

Parmi les composés de formule (VI), on peut citer la paraphénylènediamine, la p-toluylènediamine, la méthoxyparaphénylènediamine, la chloroparaphénylènediamine, la 2,3-diméthyl paraphénylènediamine, la 2,6-diméthylparaphénylènediamine, la 2,6-diéthylparaphénylènediamine, la 2,5-diméthylparaphénylène diamine, la 2-méthyl 5-méthoxyparaphénylènediamine, la 2,6-diméthyl 5-méthoxyparaphénylènediamine, la N,N-diméthylparaphénylène diamine, la N,N-diéthylparaphénylènediamine la N,N-dipropylparaphénylènediamine, la 3-méthyl 4-amino N,N-diéthylaniline, la N,N-di-(β-hydroxyéthyl)paraphénylènediamine, la 3-méthyl 4-amino N,N-di-(β-hydroxyéthyl)aniline, la 3-chloro 4-amino N,N-di-(β-hydroxy-éthyl)aniline, la 4-amino N,N-(éthylcarbamyl méthyl)aniline, la 3-méthyl 4-amino N,N-(éthyl,carbamylméthyl) aniline, la 4-amino N,N-(éthyl,β-pipéridinoéthyl)aniline, la 3-méthyl 4-amino N,N-(éthyl, β-pipéridinoéthyl)aniline, la 4-amino N,N-(éthyl, β-morpholinoéthyl)aniline, la 3-méthyl 4-amino N,N-(éthyl,β-morpholinoéthyl)aniline, la 4-amino N,N-(éthyl,β-acétylaminoéthyl) aniline, la 4-amino N-(β-méthoxyéthyl) aniline, la 3-méthyl 4-amino N,N-(éthyl,β-acétylaminoéthyl) aniline, la 4-amino N,N-(éthyl,β-mésylaminoéthyl) aniline, la 3-méthyl 4-amino N,N-(éthyl,β-mésylaminoéthyl)aniline, la 4-amino N,N-(éthyl,β-sulfoéthyl) aniline, la 3-méthyl 4-amino N,N-(éthyl,β-sulfoéthyl) aniline, la N-[(4'-amino)phényl]morpholine, la N-[(4'-amino)phényl]pipéridine, la 2-hydroxyéthylparaphénylènediamine, la fluoroparaphénylènediamine, la carboxyparaphénylènediamine, la sulfoparaphénylènediamine, la 2-isopropylparaphénylènediamine, la 2-n-propylparaphénylènediamine, l'hydroxy-2-n-propylparaphénylènediamine, la 2-hydroxyméthyl paraphénylènediamine, la N,N-diméthyl 3-méthylparaphénylène diamine, la N,N-(éthyl,β-hydroxyéthyl)paraphénylènediamine, la N-(dihydroxypropyl)paraphénylènediamine, la N-4'-aminophénylparaphénylènediamine, la N-phénylparaphénylènediamine.

Ces précurseurs de colorants d'oxydation de type para peuvent être introduits dans la composition tinctoriale, soit sous forme de base libre, soit sous forme de sels, tels que chlorhydrate, bromhydrate ou sulfate.

Parmi les p-aminophénols, on peut citer le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2,5-diméthyl 4-aminophénol, le 2-hydroxyméthyl 4-aminophénol, le 2-(β-hydroxyéthyl)4-aminophénol, le 2-méthoxy 4-aminophénol, le 3-méthoxy 4-aminophénol, le 3-(β-hydroxyéthoxy)4-aminophénol, le 2-méthoxyméthylparaaminophénol, le 2-aminométhyl 4-aminophénol, le 2-β-hydroxyéthylaminométhyl 4-aminophénol, le 2-éthoxyméthyl p-aminophénol, le 2-(β-hydroxyéthoxy)méthyl p-aminophénol.

Les bases dites doubles sont des bis-phénylalkylènediamines, répondant à la formule : 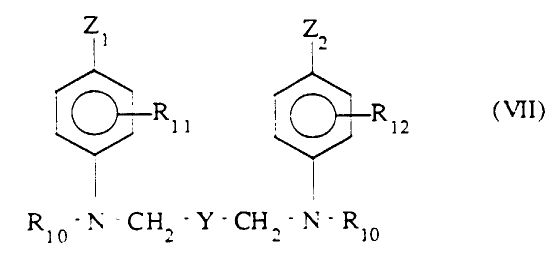 dans laquelle :
Z₁ et Z₂, identiques ou différents, représentent des groupements hydroxyle ou NHR₁₃, où R₁₃ désigne un atome d'hydrogène ou un radical alkyle inférieur ;
R₁₁ et R₁₂, identiques ou différents, représentent soit des atomes d'hydrogène, soit des atomes d'halogène, soit encore des groupements alkyle ;
R₁₀ représente un atome d'hydrogène, un groupe alkyle, hydroxyalkyle ou aminoalkyle, dont le reste amino peut être substitué;
Y représente un radical pris dans le groupe constitué par les radicaux suivants :

   -(CH₂)ₙ-, (CH₂)ₘ -O-(CH₂)ₘ,

   -(CH₂)_{q} - CHOH - (CH₂)_{q}, 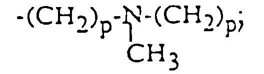
n est un nombre entier compris entre 0 et 8 et m, q et p sont des nombres entiers compris entre 0 et 4. Cette base pouvant se présenter également sous forme de ses sels d'addition avec des acides.

Les radicaux alkyle ou alcoxy ci-dessus indiqués désignent de préférence un groupement ayant 1 à 4 atomes de carbone et notamment méthyle, éthyle, propyle, méthoxy, éthoxy.

Parmi les composés de formule (VII), on peut citer le N,N'-bis-(β-hydroxyéthyl)N,N'-bis-(4'-aminophényl)]1,3-diamino 2-propanol, la N,N'-bis-(β-hydroxyéthyl)N,N'-bis-(4'-aminophényl)éthylènediamine, la N,N'-bis-(4-aminophényl)tétraméthylènediamine, la N,N'-bis-(β-hydroxyéthyl)N,N'-bis-(4-aminophényl)tétraméthylènediamine, la N,N' bis-(4-méthylaminophényl)tétraméthylènediamine, la N,N'-bis-(éthyl) N,N'-bis-(4'-amino 3'-méthylphényl)éthylènediamine.

Parmi les orthoaminophénols, on peut citer le 1-amino 2-hydrobenzène, le 6-méthyl 1-hydroxy 2-aminobenzène, le 4 méthyl 1-amino 2-hydroxybenzène, le 4-acétylamino 1-amino 2-hydroxybenzène.

Les compositions tinctoriales peuvent également contenir en plus du coupleur répondant à la formule (I) définie ci-dessus, d'autres coupleurs connus en eux-mêmes, tels que les métadiphénols, les métaaminophénols, les métaphénylènediamines, les métaacylaminophénols, les métauréidophénols, les métacarbalcoxyaminophénols, l'α-naphtol, les dérivés indoliques, les coupleurs possédant un groupement méthylène actif, tels que les composés β-cétoniques, les pyrazolones.

On peut plus particulièrement citer, à titre d'exemple le 2,4-dihydroxyphénoxyéthanol, le 2,4-dihydroxyanisole, le métaaminophénol, le monométhyléther de résorcine, la résorcine, la 2-méthylrésorcine, le 2-méthyl 5-aminophénol, le 2-méthyl 5-N-(β-hydroxy, éthyl)aminophénol, le 2-méthyl 5-N-(β-mésylaminoéthyl)aminophénol, le 2,6-diméthyl 3-aminophénol, la 6-hydroxybenzomorpholine, le 2,4-diaminoanisole, le 2,4-diaminophénoxyéthanol, la 6-amino-benzomorpholine, le [2-N-(β-hydroxyéthyl) amino 4-amino]-phénoxyéthanol, le 2-amino 4-N-(β-hydroxyéthyl) amino anisole, le (2,4-diamino)phényl-β-γ-dihydroxypropyléther, la 2,4-diamino-phénoxyéthylamine, le 1,3-diméthoxy 2,4-diaminobenzène, le 1,3,5-triméthoxy 2,4-diaminobenzène, le 1-amino 3,4-méthylènedioxybenzène, le 1-hydroxy 3,4-méthylènedioxybenzène, le 2-chloro 6-méthyl 3-aminophénol, le 2-méthyl 3-aminophénol, le 2-chlororésorcinol, la 6-méthoxy 3-hydroxyéthylaminoaniline, le 1-éthoxy 2-bis(β-hydroxyéthyl)amino 4-aminobenzène, le 3-diéthylaminophénol, le 1,3-dihydroxy 2-méthylbenzène, le 1-hydroxy 2,4-dichloro 3-aminobenzène, le 4,6-hydroxyéthoxy 1,3-diaminobenzène, le 4-méthyl 6-éthoxy 1,3-diaminobenzène, le 4-chloro 6-méthyl 3-aminophénol, le 6-chloro 3-trifluoroéthylaminophénol, et leurs sels.

On peut rajouter à ces compositions, comme cela est bien connu dans l'état de la technique, des colorants directs, tels que des colorants azoïques, anthraquinoniques ou les dérivés nitrés de la série benzénique.

L'ensemble des précurseurs de colorants par oxydation de type para et/ou ortho, ainsi que les coupleurs utilisés dans les compositions tinctoriales conformes à l'invention, représente de préférence de 0,3 à 7% en poids par rapport au poids total de la composition. La concentration en composés (I) peut varier entre de 0,05 et 3,5% en poids du poids total de la composition.

Le milieu solvant approprié pour la teinture est généralement aqueux et son pH peut varier entre 4 et 11.

Il est ajusté à la valeur désirée à l'aide d'agents alcalinisants bien connus dans l'état de la technique, tels que l'ammoniaque, les carbonates alcalins, les alcanolamines comme la mono-, la di- ou la triéthanolamine.

Les compositions tinctoriales conformes à l'invention contiennent également, dans leur forme de réalisation préférée, des agents tensio-actifs anioniques, cationiques, non-ioniques, amphotères ou leurs mélanges. Parmi ces agents tensio-actifs, on peut citer les alkylbenzènesulfonates, les alkylnaphatalènesulfonates, les sulfates, les éthersulfates et les sulfonates d'alcools gras, les sels d'ammonium quaternaires, tels que le bromure de triméthylcétylammonium, le bromure de cétylpyridinium, les éthanolamides d'acides gras éventuellement oxyéthylénés, les alcools polyglycérolés, les alkylphénols polyoxyéthylénés ou polyglycérolés, ainsi que les alkylsulfates polyoxyéthylénés.

Ces agents tensio-actifs sont présents dans les compositions conformes à l'invention dans des proportions comprises entre 0,5 et 55% en poids, et de préférence entre 2 et 50% en poids par rapport au poids total de la composition.

Ces compositions peuvent également contenir des solvants organiques pour solubiliser les composés qui ne seraient pas suffisamment solubles dans l'eau. Parmi ces solvants, on peut citer à titre d'exemple, les alcanols inférieurs en C₁-C₄, tels que l'éthanol et l'isopropanol; le glycérol; les glycols ou éthers de glycols, comme le 2-butoxyéthanol, l'éthylèneglycol, le propylèneglycol, le monoéthyl éther et le monométhyléther du diéthylèneglycol, ainsi que les alcools aromatiques comme l'alcool benzylique ou le phénoxyéthanol, les produits analogues et leurs mélanges.

Les solvants sont présents de préférence dans des proportions comprises entre 1 et 40% en poids, et en particulier entre 5 et 30% en poids par rapport au poids total de la composition.

Les agents épaississants que l'on peut ajouter dans les compositions conformes à l'invention peuvent être choisis parmi l'alginate de sodium, la gomme arabique, les dérivés de cellulose, les hétérobiopolysaccharides, tels que la gomme de xanthane, les polymères d'acide acrylique éventuellement réticulés. On peut également utiliser des agents épaississants minéraux, tels que la bentonite. Ces agents épaississants sont présents de préférence dans des proportions comprises entre 0,1 et 5%, et en particulier entre 0,2 et 3% en poids par rapport au poids total de la composition.

Les agents antioxydants qui peuvent être présents dans les compositions sont choisis en particulier parmi le sulfite de sodium. l'acide thioglycolique, le bisulfite de sodium, l'acide déhydroascorbique, l'hydroquinone et l'acide homogentisique. Ces agents antioxydants sont présents dans la composition dans des proportions comprises entre 0,05 et 1,5% en poids par rapport au poids total de la composition.

Ces compositions peuvent également contenir d'autres adjuvants cosmétiquement acceptables, tels que par exemple des agents de pénétration, des agents séquestrants, des parfums, des tampons, etc.

Les compositions conformes à l'invention peuvent se présenter sous des formes diverses, telles que sous forme de liquides, de crèmes, de gels ou sous toute autre forme appropriée pour réaliser une teinture des fibres kératiniques et notamment des cheveux humains. Ces compositions peuvent être conditionnées en flacons aérosols en présence d'un agent propulseur et former des mousses.

Les compositions tinctoriales conformes à l'invention contenant un précurseur de colorant par oxydation du type para et/ou ortho et un coupleur de formule (I), sont utilisées suivant un procédé mettant en oeuvre la révélation par un agent oxydant.

Conformément à ce procédé on mélange, au moment de l'emploi, la composition tinctoriale décrite ci-dessus avec une solution oxydante en une quantité suffisante pour pouvoir développer une coloration, le pH de la composition résultante étant inférieur à 8, puis on applique le mélange obtenu sur les fibres kératiniques et en particulier, les cheveux humains.

Le pH de la composition appliquée sur les cheveux varie de préférence entre 3,5 et 7. La solution oxydante contient à titre d'agent oxydant, l'eau oxygénée, le peroxyde d'urée, des persels, tels que le persulfate d'ammonium ou des bromates de métaux alcalins. On utilise de préférence une solution d'eau oxygénée à 20 volumes.

Le mélange obtenu est appliqué sur les cheveux et on laisse poser pendant 10 à 40 minutes, de préférence 15 à 30 minutes, après quoi on rince les cheveux, on les lave au shampooing, on les rince à nouveau et on les sèche.

Le coupleur de formule (I) définie ci-dessus, peut également être mis en oeuvre dans un procédé à plusieurs étapes, consistant dans l'une des étapes, à appliquer le précurseur de colorant d'oxydation du type para et/ou ortho ou leur mélange et, dans une autre étape, à appliquer une composition tinctoriale contenant le coupleur de formule (I).

L'agent oxydant peut être introduit, juste avant l'application, dans la composition appliquée dans le deuxième temps ou bien être appliqué sur les fibres kératiniques elles-mêmes, dans un troisième temps, les conditions de pose, de séchage et de lavage étant identiques à celles indiquées ci-dessus.

Les exemples suivants sont destinés à illustrer l'invention sans pour autant présenter un caractère limitatif.

### EXEMPLE DE PREPARATION 1

### Synthèse du 2-méthylthio 5-aminophénol.

### 1ère étape : Synthèse du 2-méthylthio 5-nitrophénol.

A une suspension de 100 g (1,42 moles) de thiométhylate de sodium dans 300 ml de N-méthylpyrrolidone agitée à température ambiante, on ajoute en 35 minutes et par portions. 167 g (1 mole) de 4-nitro 1,2-méthylènedioxybenzène de façon à maintenir la température de réaction entre 35 et 40°C. Ce chauffage est prolonté après la fin de l'addition, jusqu'à réaction complète (environ 20 minutes).

Le mélange réactionnel est coulé dans 2,2 litres d'eau glacée. La solution obtenue est neutralisée avec environ 100 ml d'acide acétique glacial.

Une huile précipite et cristallise.

On essore et lave à l'eau.

Après recristallisation de 400 ml d'acétate d'isopropyle au reflux, on obtient 148,2 g de cristaux jaunes dont le point de fusion est 145°C.

| Analyse élémentaire calculée pour C₇H₇NO₃S | | | | | |
|---|---|---|---|---|---|
| | C | H | N | O | S |
| Théorie | 45,39 | 3,81 | 7,56 | 25,92 | 17,31 |
| Trouvé | 45,51 | 3,79 | 7,61 | 26,18 | 17,38 |

### 2ème étape : Synthèse du 2-méthylthio 5-aminophénol.

On porte au reflux la suspension obtenue par mélange de 150 ml d'éthanol à 96°, 16 ml d'eau, 2,3 g de chlorure d'ammonium et 78 g de zinc en poudre fine. On ajoute par portions 25,1 g (0,15 mole) de 2-méthylthio 5-nitrophénol de façon à maintenir le reflux sans chauffage (réaction exothermique).

Le reflux est maintenu jusqu'à réaction complète (décoloration).

On filtre à chaud et lave le zinc avec le minimum d'alcool bouillant.

Le filtrat est refroidi dans un bain de glace.

Le précipité cristallisé obtenu est essoré, lavé à l'alcool et séché sous vide.

Après concentration des eaux-mères, on récupère un second jet de cristaux.

On obtient en tout 10,5 g de cristaux blancs dont le point de fusion est 175°C.

| Analyse élémentaire calculée pour C₇H₉NOS + 1/4 H₂O | | | | | |
|---|---|---|---|---|---|
| | C | H | N | O | S |
| Théorie | 52,64 | 5,99 | 8,77 | 12,52 | 20,08 |
| Trouvé | 52,91 | 5,83 | 8,91 | 12,68 | 20,10 |

### EXEMPLE DE PREPARATION 2

### Synthèse du 2-éthylthio 5-aminophénol.

### 1ère étape : Synthèse du 2-éthylthio 5-nitrophénol.

Préparé selon le mode opératoire de l'exemple 1, 1ère étape, on obtient des cristaux jaunes dont le point de fusion est 93°C (recristallisé du benzène).

| Analyse élémentaire calculée pour C₈H₉NO₃S | | | | | |
|---|---|---|---|---|---|
| | C | H | N | O | S |
| Théorie | 48,23 | 4,55 | 7,03 | 24,09 | 16,09 |
| Trouvé | 48,12 | 4,61 | 7,18 | 23,86 | 16,32 |

### 2ème étape : Synthèse du 2-éthylthio 5-aminophénol.

A une solution de 25,6 g (0,64 mole) de soude en pastilles dans 320 ml d'eau, on ajoute 100 g d'hydrosulfite de sodium technique et on porte la température à 50°C.

On ajoute par portions 39,8 g (0,2 mole) de 2-éthylthio 5-nitro phénol en maintenant la température entre 55° et 60°C.

A la fin de l'addition, on ajoute 50 ml de lessive de soude 10N et 20 g d'hydrosulfite de sodium pour rester basique et compléter la réduction.

Après 20 minutes environ à 55-60°C, on refroidit dans un bain de glace, neutralise avec de l'acide acétique glacial, essore le solide précipité et lave à l'eau.

Ce solide est dissous dans 500 ml d'acétate d'éthyle, traité avec 10 g de charbon LECA et séché sur sulfate de sodium.

Après filtration, on évapore à sec sous pression réduite et reprend dans le minimum de dichlorométhane.

Les cristaux blancs formés sont essorés (7 g) et recristallisés de l'acétonitrile; le point de fusion est de 129°C.

| Analyse élémentaire calculée pour C₈H₁₁NOS | | | | | |
|---|---|---|---|---|---|
| | C | H | N | O | S |
| Théorie | 56,77 | 6,55 | 8,28 | 9,45 | 18,95 |
| Trouvé | 56,88 | 6,54 | 8,26 | 9,62 | 18,76 |

### EXEMPLE DE PREPARATION 3

### Synthèse du 2-butylthio 5-aminophénol.

### 1ère étape : Synthèse du 2-butylthio 5-nitrophénol.

Préparé selon le mode opératoire de l'exemple 1, étape 1, on obtient des cristaux jaunes dont le point de fusion est 50°C (recristallisé du cyclohexane) et dont l'analyse élémentaire calculée pour C₁₀H₁₃NO₃S + 1/2 mole N-méthylpyrrolidone est :

| | C | H | N | O | S |
|---|---|---|---|---|---|
| Théorie | 54,23 | 6,37 | 7,59 | 20,23 | 11,58 |
| Trouvé | 54,86 | 6,31 | 7,77 | 20,40 | 11,04 |

### 2ème étape : Synthèse du 2-butylthio 5-aminophénol.

Préparé selon le mode opératoire de l'exemple 1, étape 2, on obtient des cristaux blancs dont le point de fusion est 92°C (recristallisé de l'éthanol 95°) et dont l'analyse élémentaire calculée pour C₁₀H₁₅NOS est :

| | C | H | N | O | S |
|---|---|---|---|---|---|
| Théorie | 60,88 | 7,66 | 7,10 | 8,11 | 16,25 |
| Trouvé | 60,92 | 7,71 | 7,04 | 8,30 | 16,20 |

### EXEMPLE DE PREPARATION 4

### Synthèse du 2-benzylthio 5-aminophénol.

### 1ère étape : Synthèse du 2-benzylthio 5-nitrophénol.

On chauffe à 90°C sous azote une suspension de 33,4 g (0,2 mole) de 4-nitro 1,2-méthylènedioxybenzène et 30,4 g de carbonate de potassium dans 100 ml de N-méthylpyrrolidone. En 1 heure, on coule goutte à goutte une solution de 24,8 g (0,2 mole) de benzylmercaptan dans 50 ml de N-méthylpyrrolidone.

Le mélange réactionnel est versé dans 1 litre d'eau glacée. On élimine par filtration le léger précipité cristallisé et acidifie le filtrat orangé avec de l'acide acétique glacial.

L'huile ainsi précipitée est décantée et dissoute dans 90 ml d'isopropanol.

Par addition d'eau, on précipite des cristaux jaune pâle qui sont essorés et recristallisés de 40 ml d'acétonitrile bouillant.

On obtient 18,3 g d'un composé dont le point de fusion est 103°C et dont l'analyse élémentaire calculée pour C₁₃H₁₁NO₃S + 1 mole de N-méthylpyrrolidone est :

| | C | H | N | O | S |
|---|---|---|---|---|---|
| Théorie | 59,98 | 5,59 | 7,77 | 17,76 | 8,90 |
| Trouvé | 60,11 | 5,90 | 7,76 | 17,61 | 8,81 |

### 2ème étape : Synthèse du 2-benzylthio 5-aminophénol.

La réduction est effectuée selon le mode opératoire décrit dans l'exemple 1, étape 2.

On obtient des cristaux blancs qui, après recristallisation de l'éthanol à 95° bouillant, fondent à 126°C et dont l'analyse élémentaire calculée pour C₁₃H₁₃NOS est :

| | C | H | N | O | S |
|---|---|---|---|---|---|
| Théorie | 67,50 | 5,66 | 6,06 | 6,92 | 13,86 |
| Trouvé | 67,56 | 5,66 | 5,99 | 7,06 | 13,97 |

### EXEMPLE DE PREPARATION 5

### Synthèse du 2-(β-hydroxyéthylthio)5-aminophénol.

A une solution de 35,1 g (0,45 mole) de thioéthanol dans 70 ml de N-méthylpyrrolidone, on ajoute 19,8 g de potasse en pastilles et agite jusqu'à dissolution complète (exothermique - maintenir la température à 45-50°C). Puis on ajoute en 10 minutes et par portions 33,4 g (0,2 mole) de 4-nitro 1,2-méthylènedioxybenzène.

Après 1 heure d'agitation à 50°C, la suspension violette est versée dans 400 ml d'eau glacée.

Cette solution est acidifiée avec de l'acide chlorhydrique à 36%.

L'huile orangée ainsi précipitée est décantée, lavée plusieurs fois à l'eau et est directement réduite selon le mode opératoire décrit dans l'exemple 2, deuxième étape.

Après purification par passage sur une colonne de gel de silice (éluant heptane/acétate d'éthyle), on obtient 2,5 g de cristaux blancs (recristallisés du dichloro 1,2-éthane) dont le point de fusion est 79°C et dont l'analyse élémentaire calculée pour C₈H₁₁NO₂S est :

| | C | H | N | O | S |
|---|---|---|---|---|---|
| Théorie | 51,87 | 5,99 | 7,56 | 17,27 | 17,31 |
| Trouvé | 51,91 | 6,05 | 7,54 | 17,37 | 17,32 |

### EXEMPLE DE PREPARATION 6

### Synthèse du 2-(β-acétylaminoéthylthio)5-aminophénol.

### 1ère étape : Synthèse du 2-(β-acétylaminoéthylthio)5-nitrophénol.

Ce composé est préparé selon le mode opératoire décrit dans l'exemple 5, la température de réaction étant de 70-75°C.

On obtient des cristaux jaunes (recristallisés de l'éthanol à 96°) dont le point de fusion est 204°C et dont l'analyse élémentaire calculée pour C₁₀H₁₂N₂O₄S est :

| | C | H | N | O | S |
|---|---|---|---|---|---|
| Théorie | 46,87 | 4,72 | 10,93 | 24,97 | 12,51 |
| Trouvé | 46,78 | 4,80 | 10,86 | 24,74 | 12,36 |

### 2ème étape :Synthèse du 2-(β-acétylaminoéthylthio)5-aminophénol.

La réduction du dérivé nitré correspondant est effectuée selon le procédé de l'exemple 1, étape 2. On obtient des cristaux blancs (recristallisés de l'éthanol à 95° bouillant) dont le point de fusion est de 144°C et dont l'analyse élémentaire calculée pour C₁₀H₁₁N₂O₂S + 1/4 mole d'eau est :

| | C | H | N | O | S |
|---|---|---|---|---|---|
| Théorie | 52,04 | 6,33 | 12,14 | 15,60 | 13,89 |
| Trouvé | 52,21 | 6,33 | 12,06 | 15,78 | 13,76 |

### EXEMPLE DE PREPARATION 7

### Synthèse du 2-méthylthio 5-uréidophénol.

On dissout 10,9 g (0,07 mole) du composé décrit dans l'exemple 1, étape 2, dans une solution de 7 ml d'acide chlorhydrique à 36% dans 35 ml d'eau. On ajoute en une fois et à température ambiante une solution de 6.8 g de cyanate de potassium dans 21 ml d'eau.

Après avoir agité vivement pendant 2 heures, on essore le précipité cristallisé formé, réempate dans l'eau et recristallise de l'acétonitrile bouillant.

On obtient 6,9 g de cristaux gris-violet dont le point de fusion est 136°C et dont l'analyse élémentaire calculée pour C₈H₁₀N₂O₂S est :

| | C | H | N | O | S |
|---|---|---|---|---|---|
| Théorie | 48,47 | 5,08 | 14,13 | 16,14 | 16,17 |
| Trouvé | 48,41 | 5,11 | 14,12 | 16,25 | 15,97 |

### EXEMPLE DE PREPARATION 8

### Synthèse du 2-méthylthio 5-acétylaminophénol.

On mélange 10,9 g (0,07 mole) du composé décrit dans l'exemple 1, étape 2, 25 ml de dioxane et 7,5 ml d'anhydride acétique.

Après 30 minutes de chauffage au bain-marie bouillant, le milieu réactionnel est refroidi à 5°C dans un bain de glace.

Le précipité cristallisé est essoré et recristallisé du diméthoxy-éthane bouillant.

On obtient 7,8 g de cristaux rosés dont le point de fusion est 168°C et dont l'analyse élémentaire calculée pour C₉H₁₁O₂NS est :

| | C | H | N | O | S |
|---|---|---|---|---|---|
| Théorie | 54,80 | 5,62 | 7,10 | 16,22 | 16,25 |
| Trouvé | 54,89 | 5,62 | 7,18 | 16,40 | 16,29 |

### EXEMPLE DE PREPARATION 9

### Synthèse du 2-méthylthio 5-carbéthoxyaminophénol.

On chauffe au bain-marie bouillant le mélange de 10,9 g (0,07 mole) du composé décrit dans l'exemple 1, étape 2, 4,3 g de carbonate de sodium et 35 ml de dioxane. On ajoute goutte à goutte 7,6 ml de chloroformiate d'éthyle et après 1 heure de chauffage, on verse le milieu réactionnel dans 150 ml d'eau glacée.

Le précipité obtenu, d'abord huileux, cristallise.

Après essorage, lavage à l'eau et séchage sous vide en présence d'anhydride phosphorique, on obtient 11,3 g de cristaux gris clair (recristallisés du benzène bouillant) dont le point de fusion est 95°C et dont l'analyse élémentaire calculée pour C₁₀H₁₃NO₃S est :

| | C | H | N | O | S |
|---|---|---|---|---|---|
| Théorie | 52,85 | 5,77 | 6,16 | 21,12 | 14,11 |
| Trouvé | 52,85 | 5,78 | 6,24 | 21,08 | 14,11 |

Dans le tableau (I) figurent les exemples 1 à 8 illustrant les compositions.

Dans le tableau (II) figure le pH des compositions des exemples 1 à 4, après mélange poids par poids avec une solution d'eau oxygénée à 20 volumes, dont le pH est ajusté à 3, et des exemples 5 à 8 après mélange poids pour poids avec une solution d'eau oxygénée à 20 volumes dont le pH est ajusté à 1,5.

Le mélange est appliqué sur des cheveux gris naturels à 90% de blancs ou des cheveux gris permanentés, pendant 30 minutes à température ambiante. Les cheveux sont alors rincés, lavés au shampooing, rincés une nouvelle fois, puis séchés.

Ils sont teints dans la couleur indiquée au bas du tableau (II).

### EXEMPLE DE PREPARATION 10

### Préparation du chlorhydrate de 5-amino 2,4-bis-méthylthiophénol

### 1ère étape : Synthèse du 2,4-bis-méthylthio 5-nitrophénol

A une suspension de thiométhylate de sodium (0,3 mole) dans 120 ml de diméthoxyéthane à température ambiante, on ajoute par portions 26,5 g (0,1 mole) de 5-nitro-6-(2,2,2-trifluoroéthoxy)benzo[1,3] dioxole (RN 115132-98-4).

La réaction est exothermique.

A la fin de l'addition, on chauffe une demi-heure à 60°C.

Le milieu réactionnel est versé dans 1 litre d'eau glacée (solution) et acidifié avec de l'acide acétique.

Le précipité cristallisé est essoré, réempâté dans l'eau et séché sous vide sur anhydride phosphorique.

Après recristallisation de l'acétate d'éthyle, on obtient des cristaux rouge orangé (7,8 g) fondant à 166°C et dont l'analyse élémentaire calculée pour C₈H₉NO₃S₂ est :

| | C | H | N | O | S |
|---|---|---|---|---|---|
| Théorie | 41,54 | 3,92 | 6,06 | 20,75 | 27,73 |
| Trouvé | 41,61 | 3,99 | 6,12 | 21,00 | 27,93 |

### 2ème étape : réduction

On chauffe au reflux de l'alcool un mélange de 0,8 g de chlorure d'ammonium, 3,6 ml d'eau, 70 ml d'alcool à 96° et 27 g de zinc en poudre fine.

On ajoute par portions le 2,4-bis-méthylthio 5-nitrophénol obtenu à l'étape 1 (6,9 g, 0,03 mole) de façon à maintenir le reflux sans chauffage.

La réaction est exothermique.

A la fin de l'addition, le chauffage au reflux est prolongé pendant 1 heure.

Le milieu réactionnel est filtré bouillant sur 8 ml d'alcool absolu chlorhydrique, environ 6N. Par refroidissement du filtrat, le chlorhydrate de 5-amino-2,4-bis-méthylthiophénol cristallise.

On essore, lave à l'éther éthylique et sèche sous vide sur potasse.

On obtient 4,0 g de cristaux blancs fondant avec décomposition à 182-184°C et dont l'analyse élémentaire calculée pour C₈H₁₂Cl NOS₂ est :

| | C | H | N | O | S | Cl |
|---|---|---|---|---|---|---|
| Théorie | 40,41 | 5,09 | 5,89 | 6,73 | 26,97 | 14,91 |
| Trouvé | 40,62 | 5,13 | 6,05 | 6,76 | 26,85 | 15,06 |

### EXEMPLES DE COMPOSITIONS

**TABLEAU I**

| Précurseurs de colorants | Ex 1 | Ex 2 | Ex 3 | Ex 4 | Ex 5 | Ex 6 | Ex 7 | Ex 8 |
|---|---|---|---|---|---|---|---|---|
| Paraphénylène diamine | 0,32 | | 0,32 | | | | | 0,10 |
| 2,6-diméthyl p-phénylène diamine | | 0,63g | | 0,63g | 0,63g | 0,63g | 0,63g | |
| 2-(β-acétylaminoéthylthio)5-aminophénol | 0,68 | | | | | | | |
| 2-méthylthio 5-uréidophénol | | 0,59 | | | | | | |
| 2-méthylthio 5-acétylaminophénol | | | 0,59g | | | | | |
| 2-éthylthio 5-aminophénol | | | | 0,51 g | | | | |
| 2-(β-hydroxéthylthio) 5-aminophénol | | | | | 0,55g | | | |
| 2-méthylthio 5-carbéthoxyaminophénol | | | | | | 0,68g | | |
| 2-benzylthio 5-aminophénol | | | | | | | 0,69g | |
| 2-méthylthio 5-aminophénol | | | | | | | | 0,15g |
| Support | Sup B | Sup B | Sup B | Sup B | Sup A | Sup A | Sup A | Sup A |
| pH | 9,5 | 9,1 | 9,4 | 9,1 | 9,1 | 9,1 | 9,1 | 9,5 |

**TABLEAU II**

| | Ex 1 | Ex 2 | Ex 3 | Ex 4 | Ex 5 | Ex 6 | Ex 7 | Ex 8 |
|---|---|---|---|---|---|---|---|---|
| pH mélange | 6,6 | 6,4 | 6,7 | 6,4 | 6,6 | 6,6 | 6,5 | 6,8 |
| Couleur cheveux gris naturels | châtain clair | | | blond foncé cendré | | blond clair cendré bleuté | | blond cendré nacré |
| Couleur cheveux permanentés | | blond foncé | châtain foncé cendré | | châtain clair | | blond naturel cendré | |

### EXEMPLE 9

- Chlorhydrate de 5-amino-2,4-bis-méthylthiophénol 0,71 g
- Paraphénylènediamine 0,32 g
- Support A qsp 100 g
pH = 9,1

Au moment de l'emploi, on ajoute un poids égal d'une solution d'eau oxygénée à 20 volumes dont le pH est ajusté à 1,5 par de l'acide phosphorique. Le pH du mélange est égal à 6,5.

Celui-ci est appliqué sur des cheveux gris permanentés pendant 30 minutes à température ambiante. Après rinçage, lavage au shampooing, rinçage et séchage, les cheveux sont colorés en blond naturel cendré.

### SUPPORT A

- Alcool oléique polyglycérolé à 2 moles de glycérol 4.0 g
- Alcool oléique polyglycérolé à 4 moles de glycérol à 78% de MA 5,69 g MA
- Acide oléique 3,0 g
- Amine oléique à 2 moles d'oxyde d'éthylène, vendue sous la dénomination ETHOMEEN O12 par la Société AKZO 7,0 g
- Laurylamino succinamate de diéthylaminopropyle, sel de sodium à 55% de MA 3,0 g MA
- Alcool oléique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium en solution aqueuse à 35% de MA 0,45 g MA
- Acétate d'ammonium 0,8 g
- Antioxydant, séquestrant qs
- Parfum, conservateurs qs
- Monoéthanolamine qsp pH=9.8
- Colorants x g
- Eau déminéralisée qsp 100,0 g

### SUPPORT B

- Octyldodécanol vendu sous la dénomination EUTANOL D par la Société HENKEL 8,0 g
- Acide oléique 20,0 g
- Lauryléther sulfate de monoéthanolamine, vendu sous la dénomination SIPON LM 35 par la Société HENKEL 3,0 g
- Alcool éthylique 10,0 g
- Alcool benzylique 10,0 g
- Alcool cétylstéarylique à 33 moles d'oxyde d'éthylène, vendu sous la dénomination SIMULSOL GS par la Société SEPPIC 2,4 g
- Acide éthylène diamine tétracétique 0,2 g
- Solution aqueuse de polymère constitué de motifs : 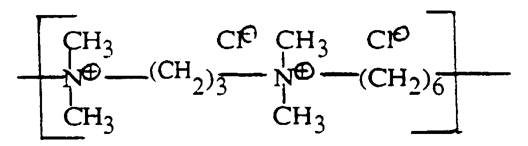 3,7 g MA
- Monoéthanolamine 7,5 g
- Diéthanolamide d'acide linoléique, vendu sous la dénomination COMPERLAN F par la Société HENKEL 8,0 g
- Ammoniaque à 20% de NH₃ 10,2 g
- Métabisulfite de sodium en solution aqueuse à 35% 1,3 g
- Hydroquinone 0,15 g
- 1-phényl 3-méthyl 5-pyrazolone 0,2 g
- Colorants x g
- Eau déminéralisée qsp 100.0 g

## Revendications

1. Utilisation d'un composé répondant à la formule : 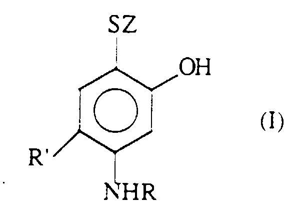 dans laquelle Z représente un radical alkyle en C₁-C₁₈, un radical aryle, aralkyle dans lequel le radical alkyle comporte 1 à 6 atomes de carbone, un radical monohydroxyalkyle en C₁-C₆, un radical polyhydroxyalkyle en C₂-C₆, un radical aminoalkyle de formule : 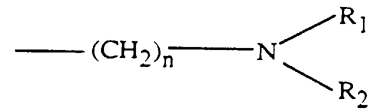 dans laquelle n est un nombre entier compris entre 1 et 6 inclus, R₁ et R₂, identiques ou différents, représentent un atome d'hydrogène ou un radical alkyle en C₁-C₄, hydroxyalkyle en C₁-C₄, acyle en C₂-C₆; R représente un atome d'hydrogène, un radical alkyle en C₁-C₆, un radical monohydroxyalkyle en C₁-C₆ ou polyhydroxyalkyle en C₂-C₆, monocarbamylalkyle en C₁-C₆, dicarbamylalkyle en C₁-C₆, aminoalkyle en C₁-C₆, acyle en C₁-C₆, carbalcoxy en C₂-C₆, carbamyle ou monoalkyle en C₁-C₆ carbamyle, R' représente un atome d'hydrogène, un radical alkyle en C₁-C₄, thioalkyle en C₁-C₄ ou alcoxy en C₁-C₄, ainsi que les sels d'acide correspondants, pour la teinture des fibres kératiniques.

2. Utilisation selon la revendication 1. caractérisée par le fait que dans le composé de formule (I) Z désigne un radical alkyle choisi parmi les radicaux méthyle, éthyle, propyle, butyle; phényle; benzyle; un radical mono- ou polyhydroxyalkyle choisi parmi les groupements -CH₂-CH₂OH, -CH₂CHOH-CH₂-OH, -CH₂CHOH-CH₃, aminoalkyle désigne -CH₂-CH₂-NH₂, -CH₂-CH₂-NHCH₃, 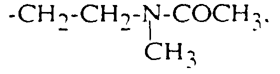 -CH₂-CH₂-NHCOCH₃,

3. Utilisation selon la revendication 1 ou 2, caractérisée par le fait que dans le composé de formule (I) le groupement R est choisi parmi les groupements formyle, acétyle et propionyle.

4. Utilisation selon la revendication 1, caractérisée par le fait que le composé est choisi parmi :
- le 2-méthylthio 5-aminophénol,
- le 2-éthylthio 5-aminophénol,
- le 2-butylthio 5-aminophénol,
- le 2-benzylthio 5-aminophénol,
- le 2-(β-hydroxyéthylthio) 5-aminophénol,
- le 2-(β-acétylaminoéthylthio) 5-aminophénol,
- le 2-méthylthio 5-uréidophénol,
- le 2-méthylthio 5-acétylaminophénol,
- le 2-méthylthio 5-carbéthoxyamino phénol,
- le 2,4-bis-méthylthio 5-aminophénol,
- le 2-(4'-amino 2'-hydroxyphényl)thio 5-aminophénol,
- le 2-(4'-nitrophényl)thio 5-aminophénol,
- le 2-(2'-aminophényl)thio 5-aminophénol.

5. Utilisation selon l'une quelconque des revendications 1 à 4, comme coupleur dans un procédé de teinture par oxydation des fibres kératiniques mettant en oeuvre des précurseurs de colorants d'oxydation para et/ou ortho.

6. Composition tinctoriale pour fibres kératiniques, en particulier pour cheveux humains, caractérisée par le fait qu'elle contient dans un milieu approprié pour la teinture de ces fibres, au moins un précurseur de colorant para et/ou ortho et au moins à titre de coupleur un métaaminophénol soufré répondant à la formule (I) : 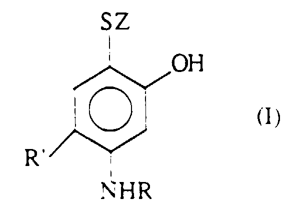 dans laquelle Z, R et R' ont les mêmes significations que dans l'une quelconque des revendications 1 à 3.

7. Composition selon la revendication 6, caractérisée par le fait que les composés de formule (I) sont choisis parmi :
- le 2-méthylthio 5-aminophénol,
- le 2-éthylthio 5-aminophénol,
- le 2-butylthio 5-aminophénol,
- le 2-benzylthio 5-aminophénol,
- le 2-(β-hydroxyéthylthio) 5-aminophénol,
- le 2-(β-acétylaminoéthylthio) 5-aminophénol,
- le 2-méthylthio 5-uréidophénol,
- le 2-méthylthio 5-acétylaminophénol,
- le 2-méthylthio 5-carbéthoxyaminophénol,
- le 2,4-bis-méthylthio 5-aminophénol,
- le 2-(4'-amino 2'-hydroxy phényl)thio 5-aminophénol,
- le 2-(4'-nitro phényl)thio 5-aminophénol,
- le 2-(2'-aminophényl)thio 5-aminophénol.

8. Composition selon l'une quelconque des revendications 6 et 7, caractérisée par le fait que les précurseurs de colorants d'oxydation para et/ou ortho sont choisis parmi les paraphénylènediamines, les paraaminophénols, les précurseurs hétérocycliques para dérivés de la pyridine ou de la pyrimidine, le 4,5-diamino 1-méthylpyrazole, les orthoamino phénols, les bis-phénylalkylènediamines.

9. Composition selon la revendication 8, caractérisée par le fait que les paraphénylènediamines sont choisies parmi les composés répondant à la formule : 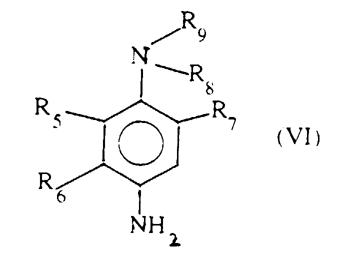 dans laquelle :
R₅, R₆, R₇, identiques ou différents, représentent un atome d'hydrogène ou d'halogène, un radical alkyle, un radical alcoxy, un radical carboxy, sulfo, hydroxyalkyle en C₁-C₄;
R₈ et R₉, identiques ou différents, représentent un atome d'hydrogène, un radidal alkyle, hydroxylkyle, alcoxyalkyle, carbamylalkyle, mésylamino alkyle, acétylaminoalkyle uréidoalkyle, carbalcoxyamino alkyle, sulfoalkyle, pipéridinoalkyle, morpholinoalkyle, phényle éventuellement substitué en para par un groupement amino ; ou bien
R₈ et R₉ forment, conjointement avec l'atome d'azote auquel ils liés, un hétérocycle pipéridino ou morpholino, sous réserve que R₅ ou R₇ représentent un atome d'hydrogène lorsque R₈ et R₉ ne représentent pas un atome d'hydrogène, ainsi que les sels de ses composés.

10. Composition selon la revendication 9, caractérisée par le fait que les paraphénylènediamines sont choisies parmi la paraphénylène diamine, la p-toluylènediamine, la chloroparaphénylènediamine, la 2,3-diméthylparaphénylènediamine, la méthoxyparaphénylènediamine, la 2,6-diméthylparaphénylènediamine, la 2,6-diéthylparaphénylène diamine, la 2,5-diméthylparaphénylènediamine, la 2-méthyl 5-méthoxyparaphénylènediamine, la 2,6-diméthyl 5-méthoxyparaphénylènediamine, la N,N-diméthylparaphénylènediamine, la N,N-diéthylparaphénylènediamine, la N,N-dipropylparaphénylènediamine, la 3-méthyl 4-amino N,N-diéthylaniline, la N,N-di-(β-hydroxyéthyl) paraphénylènediamine, la 3-méthyl 4-amino N,N-di-(β-hydroxyéthyl)aniline, la 3-chloro 4-amino N,N-di-(β-hydroxyéthyl) aniline, la 4-amino N,N-(éthyl, carbamylméthyl) aniline, la 3-méthyl 4-amino N,N-(éthyl,carbamylméthyl) aniline, la 4-amino,N,N-(éthyl,β-pipéridinoéthyl) aniline, la 3-méthyl 4-amino N,N-(éthyl, β-pipéridinoéthyl) aniline, la 4-amino N,N-(éthyl, β-morpholinoéthyl) aniline, la 3-méthyl 4-amino N,N-(éthyl β-morpholinoéthyl) aniline, la 4-amino N,N-(éthyl,β-acétylaminoéthyl) aniline, la 4-amino N-(β-méthoxyéthyl) aniline, la 3-méthyl 4-amino N,N-(éthyl,β-acétylaminoéthyl) aniline, la 4-amino N,N-(éthyl,β-mésylaminoéthyl) aniline, la 3-méthyl 4-amino N,N-(éthyl,β-mésylaminoéthyl)aniline, la 4-amino N.N-(éthyl,β-sulfoéthyl) aniline, la 3-méthyl 4-amino N,N-(éthyl,β-sulfoéthyl) aniline, la N-[(4'-amino)phényl]morpholine, la N-[(4'-amino)phényl]pipéridine, la 2-hydroxyéthylparaphénylènediamine, la fluoroparaphénylènediamine, la carboxyparaphénylènediamine, la sulfoparaphénylènediamine, la 2-isopropylparaphénylènediamine, la 2-n-propylparaphénylènedi amine, l'hydroxy-2-n-propylparaphénylène diamine, la 2-hydroxyméthylparaphénylènediamine, la N,N-diméthyl 3-méthyl p-phénylènediamine, la N,N-(éthyl,β-hydroxyéthyl)p-phénylènediamine, la N-(dihydroxypropyl)p-phénylènediamine, la N'-4'-aminophényl p-phénylènediamine, la N-phényl p-phénylène diamine.

11. Composition selon la revendication 8, caractérisée par le fait que les paraaminophénols sont choisis parmi le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2,5-diméthyl 4-aminophénol, le 2-hydroxyméthyl 4-aminophénol, le 2-(β-hydroxyéthyl)4-aminophénol, le 2-méthoxy 4-aminophénol, le 3-méthoxy 4-aminophénol, le 3-(β-hydroxyéthoxy) 4-aminophénol, le 2-aminométhyl 4-aminophénol, le 2-β-hydroxyéthyl aminométhyl 4-aminophénol, le 2-méthoxyméthyl 4-aminophénol, le 2-éthoxyméthyl 4-aminophénol, le 2- (β-hydroxyéthoxyméthyl)4-aminophénol.

12. Composition selon la revendication 8, caractérisée par le fait que les orthoaminophénols sont choisis parmi le 1-amino 2-hydroxy benzène, le 6-méthyl 1-hydroxy 2-aminobenzène, le 4-méthyl 1-amino 2-hydroxybenzène, le 4-acétylamino 1-amino 2-hydroxybenzène.

13. Composition selon la revendication 8, caractérisée par le fait que les bis-phénylalkylènediamines répondent à la formule : 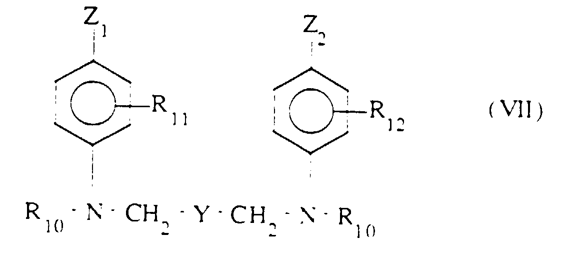 dans laquelle :
Z₁ et Z₂, identiques ou différents, représentent des groupements hydroxyle ou NHR₁₃, où R₁₃ désigne un atome d'hydrogène ou un radical alkyle inférieur ;
R₁₁ et R₁₂, indentiques ou différents, représentent soit des atomes d'hydrogène, soit des atomes d'halogène, soit encore des groupement alkyle ;
R₁₀ représente un atome d'hydrogène, un groupe alkyle, hydroxyalkyle ou aminoalkyle, dont le reste amino peut être substitué;
Y représente un radical pris dans le groupe constitué par les radicaux suivants :
-(CH₂)ₙ-, (CH₂)ₘ -O-(CH₂)ₘ,
-(CH₂)_{q} - CHOH - (CH₂)_{q}, 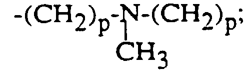
n est un nombre entier compris entre 0 et 8 et m, q et p sont des nombres entiers compris entre 0 et 4. Cette base pouvant se présenter également sous forme de ses sels d'addition avec des acides.

14. Composition selon la revendication 13, caractérisée par le fait que les bis-phénylalkylènediamines sont choisies parmi le N,N'-bis-(β-hydroxyéthyl)N,N'-bis-(4'-aminophényl)1,3-diamino 2-propanol, la N,N'-bis-(β-hydroxyéthyl)N,N'-bis-(4'-aminophényl)éthylènediamine, la N,N'-bis-(4-aminophényl)tétraméthylènediamine, la N,N'-bis-(β-hydroxyéthyl)N,N'-bis-(4-aminophényl)tétraméthylènediamine, la N,N'-bis-(4-méthylaminophényl)tétraméthylènediamine, la N,N'-bis-(éthyl)N,N'-bis-(4'-amino 3'-méthylphényl)éthylènediamine.

15. Composition selon l'une quelconque des revendications 6 à 14, caractérisée par le fait que les compositions contiennent en plus du coupleur de formule (I) définie ci-dessus, d'autres coupleurs connus en eux-mêmes, choisis parmi les métadiphénols, les métaaminophénols, les métaphénylènediamines, les métaacylaminophénols, les métauréidophénols, les métacarbalcoxyaminophénols, l'α-naphtol, les dérivés indoliques, les coupleurs possédant un groupement méthylène actif, tels que les composés β-cétoniques, les pyrazolones.

16. Composition selon la revendication 15, caractérisée par le fait que les coupleurs supplémentaires sont choisis parmi le 2,4-dihydroxyphénoxyéthanol, le 2,4-dihydroxyanisole, le métaaminophénol, le monométhyléther de résorcine, la résorcine, la 2-méthyl résorcine, le 2-méthyl 5-aminophénol, le 2-méthyl 5-N-(β-hydroxy éthyl)aminophénol, le 2-méthyl 5-N-(β-mésylaminoéthyl)aminophénol, le 2,6-diméthyl 3-aminophénol, la 6-hydroxybenzomorpholine, le 2,4-diaminoanisole, le 2,4-diamino phénoxyéthanol, la 6-aminobenzomorpholine, le [2-N-(β-hydroxyéthyl) amino 4-amino]-phénoxyéthanol, le 2-amino 4-N-(β-hydroxyéthyl) amino anisole, le (2,4-diamino)phényl-β-γ-dihydroxypropyléther, la 2,4-diaminophénoxyéthylamine, le 1,3-diméthoxy 2,4-diaminobenzène, le 1,3,5-triméthoxy 2,4-diaminobenzène, le 1-amino 3,4-méthylènedioxybenzène, le 1-hydroxy 3,4-méthylènedioxybenzène, le 2-chloro 6-méthyl 3-aminophénol, le 2-méthyl 3-aminophénol, le 2-chlororésorcinol, la 6-méthoxy 3-hydroxyéthylaminoaniline, le 1-éthoxy 2-bis(β-hydroxyéthyl )amino 4-aminobenzène, le 3-diéthylaminophénol, le 1,3-dihydroxy 2-méthylbenzène, le 1-hydroxy 2,4-dichloro 3-aminobenzène, le 4,6-hydroxyéthoxy 1,3-diaminobenzène, le 4-méthyl 6-éthoxy 1,3-diaminobenzène, le 4-chloro 6-méthyl 3-aminophénol, le 6-chloro 3-trifluoroéthylaminophénol, et leurs sels.

17. Composition selon l'une quelconque des revendications 6 à 16, caractérisée par le fait que la composition tinctoriale contient en plus des colorants directs choisis parmi les colorants azoïques, anthraquinoniques ou les dérivés nitrés de la série benzénique.

18. Composition selon l'une quelconque des revendications 6 à 17, caractérisée par le fait que les précurseurs de colorants d'oxydation para et/ou ortho ainsi que les coupleurs sont présents dans les compositions tinctoriales dans des proportions comprises entre 0,3 et 7% en poids par rapport au poids total de la composition.

19. Composition selon l'une quelconque des revendications 6 à 18, caractérisée par le fait que le composé de formule (I) est présent dans des proportions comprises entre 0,05 et 3,5% en poids par rapport au poids total de la composition.

20. Composition selon l'une quelconque des revendications 6 à 19, caractérisée par le fait que le milieu approprié pour la teinture est aqueux et que son pH est compris entre 4 et 11.

21. Procédé de teinture des fibres kératiniques, en particulier des cheveux, caractérisé par le fait que la teinture s'effectue à l'aide d'une composition présentant un pH inférieur à 8 obtenu après mélange de la composition tinctoriale définie dans l'une quelconque des revendications 6 à 20, avec une solution oxydante utilisée dans des quantités suffisantes pour pouvoir développer une coloration.

22. Procédé selon la revendication 21, caractérisé par le fait que le pH de la composition appliquée sur les fibres kératiniques, en particulier les cheveux, est compris entre 3,5 et 7.

23. Composé caractérisé par le fait qu'il répond à la formule : 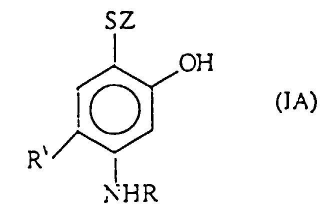 dans laquelle Z représente un radical alkyle en C₁-C₁₈, un radical aralkyle dans lequel le radical alkyle comporte 1 à 6 atomes de carbone, un radical monohydroxyalkyle en C₁-C₆, un radical polyhydroxyalkyle en C₂-C₆, un radical aminoalkyle de formule : 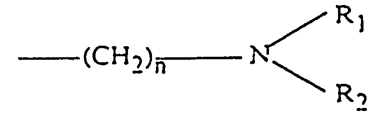 dans laquelle n est un nombre entier compris entre 1 et 6 inclus, R₁ et R₂, identiques ou différents, représentent un atome d'hydrogène ou un radical alkyle en C₁-C₄, hydroxyalkyle en C₁-C₄, acyle en C₂-C₆;
R représente un atome d'hydrogène, un radical alkyle en C₁-C₆, un radical monohydroxyalkyle en C₁-C₆ ou polyhydroxyalkyle en C₂-C₆, monocarbamylalkyle en C₁-C₆, dicarbamylalkyle en C₁-C₆ , aminoalkyle en C₁-C₆, acyle en C₁-C₆, carbalcoxy en C₂-C₆ , carbamyle ou monoalkyle en C₁-C₆ carbamyle, R' représente un atome d'hydrogène, un radical alkyle en C₁-C₄, thioalkyle en C₁-C₄ ou alcoxy en C₁-C₄, sous réserve que lorsque R et R' désignent un atome d'hydrogène, Z ne peut désigner les radicaux butyle, β,γ-dihydroxypropyle et diéthylaminoéthyle, ainsi que les sels d'acide correspondants.

24. Procédé de préparation des composés selon la revendication 23, caractérisé par le fait que l'on fait réagir en présence d'une base, le 3,4-méthylènedioxy 1-nitrobenzène éventuellement substitué en 6 par alkyle, thioalkyle ou alcoxy sur un thiol de formule (II) :
Z-SH (II)
dans laquelle Z représente un groupement alkyle en C₁-C₁₈, un groupement aralkyle dans lequel le groupement alkyle est en C₁-C₆, un groupement alkyle en C₁-C₆ monohydroxylé ou un groupement alkyle polyhydroxylé en C₂-C₆, un groupement de formule : 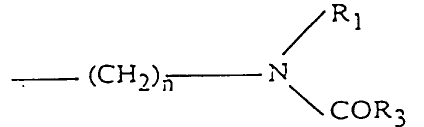 où R₁ et n ont les significations indiquées ci-dessus et où R₃ représente un atome d'hydrogène ou un radical alkyle ayant 1 à 3 atomes de carbone ; dans une deuxième étape, on réduit le groupement NO₂ du composé de formule (III) : 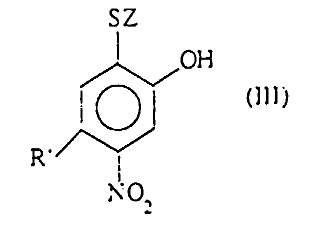 obtenu précédemment pour préparer un composé répondant à la formule (IV) : 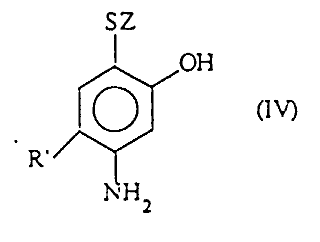 dans laquelle Z a la signification indiquée ci-dessus ; et que l'on procède, si nécessaire, dans une troisième étape, à la transformation du composé de formule (IV), soit par hydrolyse acide, lorsque Z comprend un groupement amine acylé, soit par substitution de l'amine extra-nucléaire lorsque Z comporte un groupement amine, soit par mono-substitution de l'amine aromatique pour obtenir les composés répondant à la formule (I A)

## Patentansprüche

1. Verwendung einer Verbindung der Formel: 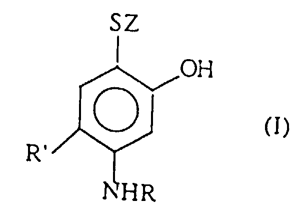 worin gilt:
Z stellt einen C₁₋₁₈-Alkyl-, Aryl-, Aralkylrest, worin der Alkylrest 1 bis 6 Kohlenstoffatome aufweist, einen Monohydroxyalkylrest mit 1 bis 6 Kohlenstoffatomen oder einen C₂₋₆-Polyhydroxyalkylrest und einen Aminoalkylrest der Formel dar: 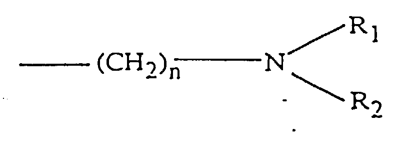
worin n eine ganze Zahl von 1 bis 6 ist und R₁ und R₂, gleich oder verschieden, ein Wasserstoffatom oder einen C₁₋₄-Alkyl-, C₁₋₄-Hydroxyalkyl- und einen C₂₋₆-Acylrest darstellen;
R stellt ein Wasserstoffatom, einen C₁₋₆-Alkyl-, C₁₋₆-Monohydroxyalkyl-, C₂₋₆-Polyhydroxyalkyl-, C₁₋₆-Monocarbamyl-, C₁₋₆-Dicarbamylalkyl-, C₁₋₆-Aminoalkyl-, C₁₋₆-Acyl-, C₂₋₆-Carbalkoxy-, Carbamyl- oder C₁₋₆-Monoalkylcarbamylrest dar; und R' stellt ein Wasserstoffatom, einen C₁₋₄-Alkyl-, C₁₋₄-Thioalkyl- oder einen C₁₋₄-Alkoxyrest dar,
sowie der entsprechenden Säuresalze zur Färbung keratinischer Fasern.

2. Verwendung gemäß Anspruch 1,
dadurch **gekennzeichnet**, daß
in der Verbindung der Formel (I) Z einen Alkylrest, ausgewählt aus Methyl-, Ethyl-, Propyl- und Butylresten, einen Phenyl-, Benzyl-, Mono- oder einen Polyhydroxyalkylrest, ausgewählt aus den Gruppierungen: -CH₂-CH₂OH, -CH₂CHOH-CH₂-OH oder -CH₂CHOH-CH₃, oder einen Aminoalkylrest bedeutet, ausgewählt aus; -CH₂-CH₂-NH₂, -CH₂-CH₂-NHCH₃; CH₂CH₂-N-CH₃COCH₃, -CH₂-CH₂-NHCOCH₃.

3. Verwendung gemäß Anspruch 1 oder 2,
dadurch **gekennzeichnet**, daß
in der Verbindung der Formel (I) die Gruppe R aus Formyl-, Acetyl- und Propionylgruppen ausgewählt ist.

4. Verwendung gemäß Anspruch 1,
dadurch **gekennzeichnet**, daß
die Verbindung ausgewählt ist aus:
- 2-Methylthio-5-aminophenol,
- 2-Ethylthio-5-aminophenol,
- 2-Butylthio-5-aminophenol,
- 2-Benzylthio-5-aminophenol,
- 2-(β-Hydroxyethylthio)-5-aminophenol,
- 2-(β-Acetylaminoethylthio)-5-aminophenol,
- 2-Methylthio-5-ureidophenol,
- 2-Methylthio-5-acetylaminophenol,
- 2-Methylthio-5-carbethoxyaminophenol,
- 2,4-Bismethylthio-5-aminophenol
- 2-(4'-Amino-2'-hydroxyphenyl)thio-5-aminophenol,
- 2-(4'-Nitrophenyl)thio-5-aminophenol,
- 2-(2'-Aminophenyl)thio-5-aminophenol.

5. Verwendung gemäß einem der Ansprüche 1 bis 4 als Kuppler in einem Verfahren zur Oxidationsfärbung keratinischer Fasern, wobei man Oxidationsfarbstoff-Vorstufenverbindungen vom p- und/oder o-Typ zur Anwendung bringt.

6. Färbezusammensetzung für keratinische Fasern, insbesondere für menschliche Haare,
dadurch **gekennzeichnet**, daß
sie in einem zur Färbung dieser Fasern geeigneten Medium mindestens eine Farbstoff-Vorstufenverbindung vom p- und/oder o-Typ und mindestens als Kuppler ein schwefelhaltiges m-Aminophenol der Formel (I) enthält: 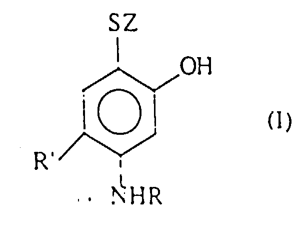 worin Z, R und R' die in jedem der Ansprüche 1 bis 3 angegebenen Bedeutungen haben.

7. Zusammensetzung gemäß Anspruch 6,
dadurch **gekennzeichnet**, daß
die Verbindungen der Formel (I) ausgewählt sind aus:
- 2-Methylthio-5-aminophenol,
- 2-Ethylthio-5-aminophenol,
- 2-Butylthio-5-aminophenol,
- 2-Benzylthio-5-aminophenol,
- 2-(β-Hydroxyethylthio)-5-aminophenol,
- 2-(β-Acetylaminoethylthio)-5-aminophenol,
- 2-Methylthio-5-ureidophenol,
- 2-Methylthio-5-acetylaminophenol,
- 2-Methylthio-5-carbethoxyaminophenol,
- 2,4-Bismethylthio-5-aminophenol
- 2-(4'-Amino-2'-hydroxyphenyl)thio-5-aminophenol,
- 2-(4'-Nitrophenyl)thio-5-aminophenol,
- 2-(2'-Aminophenyl)thio-5-aminophenol,

8. Zusammensetzung gemäß einem der Ansprüche 6 und 7,
dadurch **gekennzeichnet**, daß
die Oxidationsfarbstoff-Vorstufenverbindungen von p- und o-Typ aus p-Phenylendiaminen, p-Aminophenolen, heterozyklischen Vorstufenverbindungen von para-Derivaten des Pyridins oder Pyrimidins, aus 4,5-Diamino-1-methylpyrazol, o-Aminophenolen und aus Bisphenylalkylendiaminen ausgewählt sind.

9. Zusammensetzung gemäß Anspruch 8,
dadurch **gekennzeichnet**, daß
die p-Phenylendiamine aus Verbindungen der Formel: 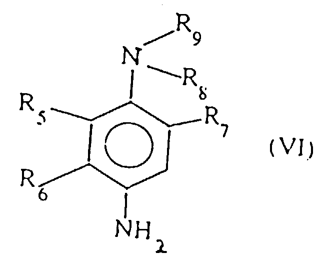 worin gilt:
R₅, R₆ und R₇ stellen, gleich oder verschieden, ein Wasserstoff- oder Halogenatom, einen Alkyl-, Alkoxy-, Carboxy-, Sulfo- oder einen C₁₋₄-Hydroxyalkylrest dar;
R₈ und R₉ stellen, gleich oder verschieden, ein Wasserstoffatom, einen Alkyl-, Hydroxyalkyl-, Akoxyalkyl-, Carbamylalkyl-, Mesylaminoalkyl-, Acetylaminoalky-, Ureidoalkyl-, Carbalkoxyaminoalkyl-, Sulfoalkyl-, Piperidinoalkyl-, Morpholinoalky- oder einen Phenylrest dar, der gegebenenfalls in p-Position mit einer Aminogruppe subsituiert ist; oder R₈ und R₉ bilden, zusammen mit dem Stickstoffatom, an das sie gebunden sind, auch einen Piperidino- oder Morpholino-Heterozyklus, mit der Maßgabe, daß R₅ oder R₇ ein Wasserstoffatom darstellen, wenn R₈ und R₉ kein Wasserstoffatom darstellen,
sowie aus den Salzen dieser Verbindungen ausgewählt sind.

10. Zusammensetzung gemäß Anspruch 9,
dadurch **gekennzeichnet**, daß
die p-Phenylendiamine ausp-Phenylendiamin, p-Toluylendiamin, Methoxy-p-phenylendiamin, Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, N,N-Diethyl-p-phenylendiamin, N,N-Dipropyl-p-phenylendiamin, 3-Methyl-4-amino-N,N-diethylanilin, N,N-Di(β-hydroxyethyl)-p-phenylendiamin, 3-Methyl-4-amino-N,N-di(β-hydroxyethyl)anilin, 3-Chlor-4-amino-N,N-di(β-hydroxyethyl)anilin, 4-Amino-(N,N-ethyl,carbamylmethyl)anilin, 3-Methyl-4-amino-N,N-(ethyl, carbamylmethyl)anilin, 4-Amino-N,N-(ethyl, β-piperidinoethyl)anilin, 4-Amino-N,N-(ethyl,β-morpholinoethyl)anilin, 3-Methyl-4-amino-N,N-(ethyl,β-morpholinoethyl)anilin, 4-Amino-N,N-(ethyl,β-acetylaminoethyl)anilin, 4-Amino-N-(β-methoxyethyl)anilin, 3-Methyl-4-amino-N,N-(ethyl,β-acetylaminoethyl)anilin, 4-Amino-N,N-(ethyl,β-mesylaminoethyl)anilin, 3-Methyl-4-amino-N,N-(ethyl,β-mesylaminoethyl)anilin, 4-Amino-N,N-(ethyl,β-sulfoethyl)anilin, 3-Methyl-4-amino-N,N-(ethyl,β-sulfoethyl)anilin, N- ((4'-Amino)phenyl)morpholin, N- ((4'-Amino)phenyl)piperidin, 2-Hydroxyethyl-p-phenylendiamin, Fluor-p-phenylendiamin, Carboxy-p-phenylendiamin, Sulfo-p-phenylendiamin, 2-Isopropyl-p-phenylendiamin, 2-n-Propyl-p-phenylendiamin, Hydroxy-2-n-propyl-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(Dihydroxypropyl)-p-phenylendiamin, N-4'-Aminophenyl-p-phenylendiamin und aus N-Phenyl-p-phenylendiamin ausgewählt sind.

11. Zusammensetzung gemäß Anspruch 8,
dadurch **gekennzeichnet**, daß
die p-Aminophenole aus p-Aminophenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2,5-Dimethyl-4-aminophenol, 2-Hydroxymethyl-4-aminophenol, 2-(β-Hydroxyethyl)-4-aminophenol, 2-Methoxy-4-aminophenol, 3-Methoxy-4-aminophenol, 3-(β-Hydroxyethoxy)-4-aminophenol, 2-Aminomethyl-4-aminophenol, 2-β-Hydroxyethylaminomethyl-4-aminophenol, 2-Methoxymethyl-4-aminophenol, 2-Ethoxymethyl-4-aminophenol und aus 2-(β-Hydroxyethoxymethyl-4-aminophenol ausgewählt sind.

12. Zusammensetzung gemäß Anspruch 8,
dadurch **gekennzeichnet**, daß
die o-Aminophenole aus 1-Amino-2-hydroxybenzol, 6-Methyl-1-hydroxy-2-aminobenzol, 4-Methyl-1-amino-2-hydroxybenzol und aus 4-Acetylamino-1-amino-2-hydroxybenzol ausgewählt sind.

13. Zusammensetzung gemäß Anspruch 8,
dadurch **gekennzeichnet**, daß
die Bisphenylalkylendiamine die Formel aufweisen: 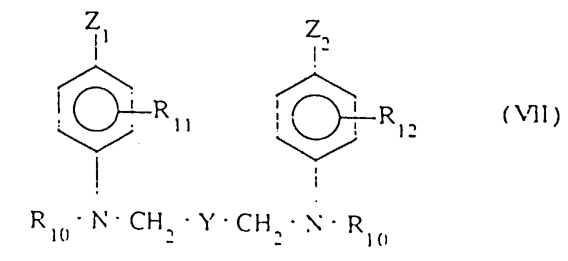 worin gilt:
Z₁ und Z₂ stellen, gleich oder verschieden, Hydroxyl- oder NHR₁₃-Gruppen dar, worin R₁₃ ein Wasserstoffatom oder einen Niedrigalkylrest bedeutet;
R₁₁ und R₁₂ stellen, gleich oder verschieden, entweder Wasserstoffatome oder Halogenatome oder auch Alkylgruppen dar;
R₁₀ stellt ein Wasserstoffatom, eine Alkyl-, Hydroxyalkyl- oder Aminoalkylgruppe dar, worin der Aminorest substituiert sein kann;
Y stellt einen Rest aus der Gruppe aus den folgenden Resten dar:
-(CH₂)ₙ-, (CH₂)ₘ -O-(CH₂)ₘ,
- (CH₂)_{q} - CHOH - (CH₂)_{q}, 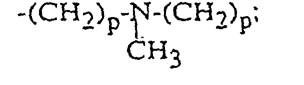
worin n eine ganze Zahl von 0 bis 8 und m, q und p ganze Zahlen von 0 bis 4 sind,
wobei diese Base auch in Form ihrer Additionssalze mit Säuren vorliegen kann.

14. Zusammensetzung gemäß Anspruch 13,
dadurch **gekennzeichnet**, daß
die Bisphenylalkylendiamine aus N,N'-Bis(β-hydroxyethyl)-N,N'-bis(4'-aminophenyl)-1,3-diaminopropan-2-ol, N,N'-Bis(β-hydroxyethyl)-N,N'-bis(4'-aminophenyl)ethylendiamin, N,N'-Bis(4-aminophenyl)tetramethylendiamin, N,N'-Bis(β-hydroxyethyl)-N,N'-bis(4-aminophenyl)tetramethylendiamin, N,N'-Bis(4-methylaminophenyl)tetramethylendiamin und aus N,N'-Bis(ethyl)-N,N'-bis(4'-amino-3'-methylphenyl)ethylendiamin ausgewählt sind.

15. Zusammensetzung gemäß einem der Ansprüche 6 bis 14,
dadurch **gekennzeichnet**, daß
die Zusammensetzungen zusätzlich zum Kuppler der oben definierten Formel (I) weitere an sich bekannte Kuppler enthalten, die aus m-Diphenolen, m-Aminophenolen, m-Phenylendiaminen, m-Acylaminophenolen, m-Ureidophenolen, m-Carbalkoxyaminophenolen, α-Naphthol, Indolderivaten, aus Kupplern mit einer aktiven Methylengruppe, wie aus β-Ketoverbindungen, sowie aus Pyrazolonen ausgewählt sind.

16. Zusammensetzung gemäß Anspruch 15,
dadurch **gekennzeichnet**, daß
die zusätzlichen Kuppler aus 2,4-Dihydroxyphenoxyethanol, 2,4-Dihydroxyanisol, m-Aminophenol, Resorcinmonomethylether, Resorcin, 2-Methylresorcin, 2-Methyl-5-aminophenol, 2-Methyl-5-N-(β-hydroxyethyl)aminophenol, 2-Methyl-5-N-(β-mesylaminoethyl)aminophenol, 2,6-Dimethyl-3-aminophenol, 6-Hydroxybenzomorpholin, 2,4-Diaminoanisol, 2,4-Diaminophenoxyethanol, 6-Aminobenzomorpholin, 2-N-(β-Hydroxyethyl)amino-4-amino)phenoxyethanol, 2-Amino-4-N-(β-hydroxyethyl)aminoanisol, (2,4-Diamino)phenyl-β,γ-dihydroxypropylether, 2,4-Diaminophenoxyethylamin, 1,3-Dimethoxy-2,4-diaminobenzol, 1,3,5-Trimethoxy-2,4-diaminobenzol, 1-Amino-3,4-methylendioxybenzol, 1-Hydroxy-3,4-methylendioxybenzol, 2-Chlor-6-methyl-3-aminophenol, 2-Methyl-3-aminophenol, 2-Chlorresorcinol, 6-Methoxy-3-hydroxyethylaminoanilin, 1-Ethoxy-2-bis(β-hydroxyethyl)amino-4-aminobenzol, 3-Diethylaminophenol, 1,3-Dihydroxy-2-methylbenzol, 1-Hydroxy-2,4-dichlor-3-aminobenzol, 4,6-Hydroxyethoxy-1,3-diaminobenzol, 4-Methyl-6-ethoxy-1,3-diaminobenzol, 4-Chlor-6-methyl-3-aminophenol, 6-Chlor-3-trifluorethylaminophenol und aus deren Salze ausgewählt sind.

17. Zusammensetzung gemäß einem der Ansprüche 6 bis 16,
dadurch **gekennzeichnet**, daß
die Färbezusammensetzung zusätzlich Direktfarbstoffe enthält, die aus Azo- und Antrachinonfarbstoffen oder nitrierten Derivaten der Benzolreihe ausgewählt sind.

18. Zusammensetzung gemäß einem der Ansprüche 6 bis 17,
dadurch **gekennzeichnet**, daß
die Oxidationsfarbstoff-Vorstufenverbindungen vom p- und/oder o-Typ sowie die Kuppler in den Färbezusammensetzungen in Mengenanteilen von 0,3 bis 7 Gew.% vorhanden sind, bezogen auf das Gesamtgewicht der Zusammensetzung.

19. Zusammensetzung gemäß einem der Ansprüche 6 bis 18,
dadurch **gekennzeichnet**, daß
die Verbindung der Formel (I) in Mengenanteilen von 0,05 bis 3,5 Gew.% vorhanden ist, bezogen auf das Gesamtgewicht der Zusammensetzung.

20. Zusammensetzung gemäß einem der Ansprüche 6 bis 19,
dadurch **gekennzeichnet**, daß
das zur Färbung geeignete Medium wässrig ist und sein pH-Wert 4 bis 11 beträgt.

21. Verfahren zur Färbung keratinischer Fasern, insbesondere der Haare,
dadurch **gekennzeichnet**, daß
die Färbung mit einer Zusammensetzung durchgeführt wird, die einen pH-Wert von weniger als 8 aufweist, welche nach Vermischung der in jedem der Ansprüche 6 bis 10 definierten Färbezusammensetzung mit einer oxidierenden Lösung erhältlich ist, die wiederum in Mengen angewandt wird, die ausreichen, um eine Färbung zu entwickeln.

22. Verfahren gemäß Anspruch 21,
dadurch **gekennzeichnet**, daß
der pH-Wert der auf die keratinischen Fasern, insbesondere die Haare, aufgebrachten Zusammensetzung 3,5 bis 7 beträgt.

23. Verbindung,
dadurch **gekennzeichnet**, daß
sie die Formel aufweist: 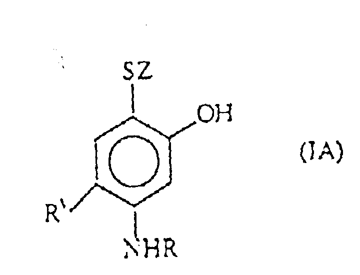 worin gilt:
Z stellt einen C₁₋₁₈-Alkylrest, einen Aralkylrest, worin der Alkylrest 1 bis 6 Kohlenstoffatome aufweist, einen C₁₋₆-Monohydroxyalkyl-, C₂₋₆-Polyhydroxyalkyl- und einen Aminoalkylrest der Formel dar: 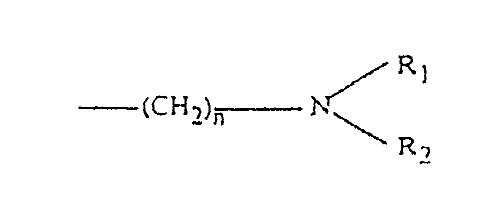
worin n eine ganze Zahl von 1 bis 6 ist und R₁ und R₂, gleich oder verschieden, ein Wasserstoffatom oder einen C₁₋₄-Alkyl-, C₁₋₄-Hydroxyalkyl- und einen C₂₋₆-Acylrest darstellen,
R stellt ein Wasserstoffatom, einen C₁₋₆-Alkyl-, C₁₋₆-Monohydroxyalkyl-, C₂₋₆-Polyhydroxyalkyl-, C₁₋₆-Monocarbamylalkyl-, C₁₋₆-Dicarbamylalkyl-, C₁₋₆-Aminoalkyl-, C₁₋₆-Acyl-, C₂₋₆-Carbalkoxy-, Carbamyl- oder einen C₁₋₆-Monoalkylcarbamylrest dar, und R' stellt ein Wasserstoffatom, einen C₁₋₄-Alkyl-, C₁₋₄-Thioalkyl- oder einen C₁₋₄-Akoxyrest dar, mit der Maßgabe, daß, wenn R und R' ein Wasserstoffatom bedeuten, Z keinen Butyl-, β,γ-Dihydroxypropyl- und Diethylaminoethylrest bedeutet,
wobei die entsprechenden Säuresalze eingeschlossen sind.

24. Verfahren zur Herstellung von Verbindungen gemäß Anspruch 23,
dadurch **gekennzeichnet**, daß
man in Gegenwart einer Base 3,4-Methylendioxy-1-nitrobenzol, das gegebenenfalls in Position 6 mit einem Alkyl-, Thioalkyl- oder Alkoxyrest substituiert ist, mit einem Thiol der Formel (II) reagieren läßt:
Z - SH (II)
worin Z entweder eine C₁₋₁₈-Alkylgruppe, eine Aralkylgruppe, worin der Alkylrest 1 bis 6 Kohlenstoffatome aufweist, oder eine Monohydroxyalkylgruppe mit 1 bis 6 Kohlenstoffatomen, eine C₂₋₆-Polyhydroxyalkylgruppe oder eine Gruppe der Formel darstellt: 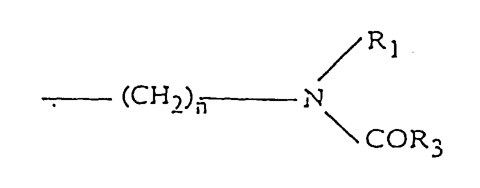
worin R₁ und n die oben angegebenen Bedeutungen haben, und worin R₃ ein Wasserstoffatom oder einen Alkylrest mit 1 bis 3 Kohlenstoffatomen darstellt, und wobei man in einer zweiten Stufe die NO₂-Gruppe der vorab erhaltenen Verbindung der Formel (III) reduziert: 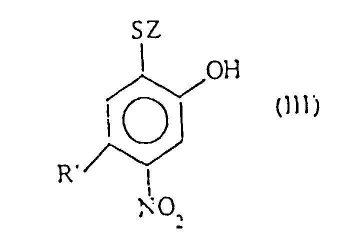 um eine Verbndung der Formel (IV) herzustellen: 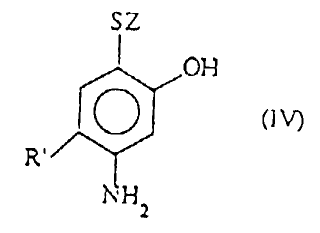
worin Z und R' die oben angegebene Bedeutung haben, und daß man gegebenenfalls in einer driten Stufe die Verbindung der Formel (V) entweder durch saure Hydrolyse, wenn Z eine acylierte Amingruppe umfaßt, oder durch Substitution an der außerhalb des Kerns befindlichen Amingruppe, wenn Z eine Amingruppe umfaßt, oder durch Monosubstitution an der aromatischen Amingruppe umsetzt,
um die Verbindungen der Formel (IA) zu erhalten.

## Claims

1. Use of a compound corresponding to the formula: 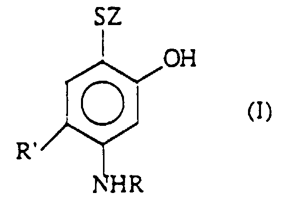 in which Z represents a C₁-C₁₈ alkyl radical, an aryl radical, an aralkyl radical in which the alkyl radical contains 1 to 6 carbon atoms, a C₁-C₆ monohydroxyalkyl radical, a C₂-C₆ polyhydroxyalkyl radical, an aminoalkyl radical of formula: 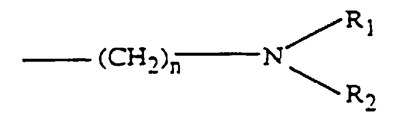 in which n is an integer between 1 and 6 inclusive, R₁ and R₂, which may be identical or different, represent a hydrogen atom or a C₁-C₄-alkyl, C₁-C₄ hydroxyalkyl, C₂-C₆ acyl radical;
R represents a hydrogen atom, a C₁-C₆ alkyl radical, a C₁-C₆ monohydroxyalkyl, C₂-C₆ polyhydroxyalkyl, monocarbamyl (C₁-C₆ alkyl), dicarbamyl (C₁-C₆ alkyl), C₁-C₆ aminoalkyl, C₁-C₆ acyl, C₂-C₆ carbalkoxy, carbamyl or mono (C₁-C₆ alkyl) carbamyl radical, R' represents a hydrogen atom, a C₁-C₄ alkyl, C₁-C₄ thioalkyl or C₁-C₄ alkoxy radical, as well as the corresponding salts with an acid, for the dyeing of keratinous fibres.

2. Use according to Claim 1, characterized in that, in the compound of formula (I), Z denotes an alkyl radical chosen from methyl, ethyl, propyl, butyl radicals; phenyl; benzyl; a mono- or polyhydroxy alkyl radical chosen from the groups -CH₂-CH₂OH, -CH₂CHOH-CH₂-OH, -CH₂CHOH-CH₃,
aminoalkyl denotes -CH₂-CH₂-NH₂,
-CH₂-CH₂-NHCH₃, 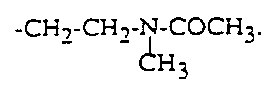
-CH₂-CH₂-NHCOCH₃;

3. Use according to Claim 1 or 2, characterized in that in the compound of formula (I), the group R is chosen from formyl, acetyl and propionyl groups.

4. Use according to Claim 1, characterized in that the compound is chosen from
- 2-methylthio-5-aminophenol,
- 2-ethylthio-5-aminophenol,
- 2-butylthio-5-aminophenol,
- 2-benzylthio-5-aminophenol,
- 2-(β-hydroxyethylthio)-5-aminophenol,
- 2-(β-acetylaminoethylthio)-5-aminophenol,
- 2-methylthio-5-ureidophenol,
- 2-methylthio-5-acetylaminophenol,
- 2-methylthio-5-(carbethoxyamino)phenol,
- 2,4-bis(methylthio)-5-aminophenol,
- 2-(4'-amino-2'-hydroxyphenyl)thio-5-aminophenol,
- 2-(4'-nitrophenyl)thio-5-aminophenol,
- 2-(2'-aminophenyl)thio-5-aminophenol.

5. Use according to any one of Claims 1 to 4, as a coupler in a process for the oxidation dyeing of keratinous fibres employing para and/or ortho oxidation dye precursors.

6. Dyeing composition for keratinous fibres, especially for human hair, characterized in that it contains, in a medium suitable for the dyeing of these fibres, at least one para and/or ortho dye precursor and at least, as a coupler, one sulphur-containing meta-aminophenol corresponding to the formula (I): 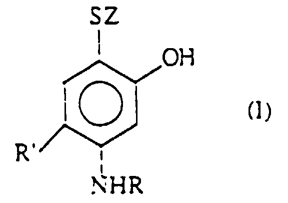 in which Z, R and R' have the same meanings as in any one of Claims 1 to 3.

7. Composition according to Claim 6, characterized in that the compounds of formula (I) are chosen from:
- 2-methylthio-5-aminophenol,
- 2-ethylthio-5-aminophenol,
- 2-butylthio-5-aminophenol,
- 2-benzylthio-5-aminophenol,
- 2-(β-hydroxyethylthio)-5-aminophenol,
- 2-(β-acetylaminoethylthio)-5-aminophenol,
- 2-methylthio-5-ureidophenol,
- 2-methylthio-5-acetylaminophenol,
- 2-methylthio-5-carbethoxyaminophenol,
- 2,4-bis(methylthio)-5-aminophenol,
- 2-(4'-amino-2'-hydroxyphenyl)thio-5-aminophenol,
- 2-(4'-nitrophenyl)thio-5-aminophenol,
- 2-(2'-aminophenyl)thio-5-aminophenol.

8. Composition according to either of Claims 6 and 7, characterized in that the para and/or ortho oxidation dye precursors are chosen from para-phenylene diamines, para-aminophenols, heterocyclic para precursors derived from pyridine or from pyrimidine, 4,5-diamino-1-methylpyrazole, ortho-aminophenols, bis(phenyl)alkylenediamines.

9. Composition according to Claim 8, characterized in that the para-phenylenediamines are chosen from the compounds corresponding to the formula: 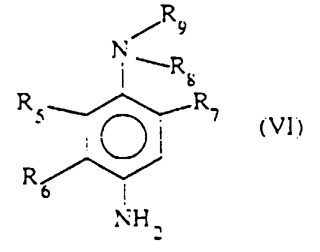 in which:
R₅, R₆, R₇, which may be identical or different, represent a hydrogen or halogen atom, an alkyl radical, an alkoxy radical, a carboxyl, sulpho, C₁-C₄ hydroxyalkyl radical; R₈ and R₉, which may be identical or different, represent a hydrogen atom, an alkyl, hydroxyalkyl, *r* alkoxyalkyl, carbamylalkyl, mesylaminoalkyl, acetylaminoalkyl, ureidoalkyl, carbalkoxyaminoalkyl, sulphoalkyl, piperidinoalkyl, morpholinoalkyl radical, a phenyl radical optionally substituted at the para position with an amino group; or alternatively R₈ and R₉, together with the nitrogen atom to which they are linked, form a piperidino or morpholino heterocycle, with the proviso that R₅ or R₇ represent [sic] a hydrogen atom when R₈ and R₉ do not represent a hydrogen atom, as well as the salts of its [sic] compounds.

10. Composition according to Claim 9, characterized in that the para-phenylenediamines are chosen from paraphenylenediamine, p-toluylenediamine, chloro-paraphenylenediamine, 2,3-dimethyl-para-phenylenediamine, methoxy-para-phenylenediamine, 2,6-dimethyl-para-phenylenediamine, 2,6-diethyl-para-phenylenediamine, 2,5-dimethyl-para-phenylenediamine, 2-methyl-5-methoxy-paraphenylenediamine, 2,6-dimethyl-5-methoxy-paraphenylenediamine, N,N-dimethyl-para-phenylenediamine, N,N-diethyl-para-phenylenediamine, N,N-dipropyl-paraphenylenediamine, 3-methyl-4-amino-N,N-diethylaniline, N,N-di(β-hydroxyethyl)-para-phenylenediamine, 3-methyl-4-amino-N,N-di(β-hydroxyethyl) aniline, 3-chloro-4-amino-N,N-di(β-hydroxyethyl)aniline, 4-amino-N,N-(ethyl,-carbamylmethyl)aniline, 3-methyl-4-amino-N,N-(ethyl,-carbamylmethyl) aniline, 4-amino-N,N-(ethyl,β-piperidinoethyl)aniline,3-methyl-4-amino-N,N- (ethyl, β-piperidinoethyl) aniline, 4-amino-N,N-(ethyl, β-morpholinoethyl)-aniline, 3-methyl-4-amino-N,N-(ethyl,β-morpholinoethyl)-aniline, 4-amino-N,N-(ethyl, β-acetylaminoethyl)aniline, 4-amino-N-(β-methoxyethyl) aniline, 3-methyl-4-amino-N,N-(ethyl,β-acetylaminoethyl)aniline, 4-amino-N,N-(ethyl,-β-mesylaminoethyl)aniline, 3-methyl-4-amino-N,N-(ethyl,β-mesylaminoethyl)aniline, 4-amino-N,N-(ethyl,β-sulphoethyl)aniline, 3-methyl-4-amino-N,N-(ethyl,β-sulphoethyl)aniline, N- [(4'-amino)phenyl]-morpholine [sic], N-[(4'-amino)phenyl]piperidine [sic] , 2-hydroxyethyl-p-phenylenediamine, fluoro-para-phenylenediamine, carboxy-para-phenylenediamine, sulpho-paraphenylenediamine, 2-isopropyl-para-phenylenediamine, 2-n-propyl-para-phenylenediamine, hydroxy-2-n-propyl-paraphenylenediamine, 2-hydroxymethyl-para-phenylenediamine, N,N-dimethyl-3-methyl-p-phenylenediamine, N,N-(ethyl,β-hydroxyethyl)-p-phenylenediamine, N-(dihydroxypropyl)-p-phenylenediamine, N'-4'-aminophenyl-p-phenylenediamine [sic], N-phenyl-p-phenylenediamine.

11. Composition according to Claim 8, characterized in that the para-aminophenols are chosen from p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2,5-dimethyl-4-aminophenol, 2-hydroxymethyl-4-aminophenol, 2-(β-hydroxyethyl)-4-aminophenol, 2-methoxy-4-aminophenol, 3-methoxy-4-aminophenol, 3-(β-hydroxyethoxy)-4-aminophenol, 2-aminomethyl-4-aminophenyl, 2-β-hydroxyethylaminomethyl-4-aminophenol [sic], 2-methoxymethyl-4-aminophenol, 2-ethoxymethyl-4-aminophenol, 2-(β-hydroxyethoxymethyl)-4-aminophenol.

12. Composition according to Claim 8, characterized in that the ortho-aminophenols are chosen from 1-amino-2-hydroxybenzene, 6-methyl-1-hydroxy-2-aminobenzene, 4-methyl-1-amino-2-hydroxybenzene, 4-acetylamino-1-amino-2-hydroxybenzene.

13. Composition according to Claim 8, characterized in that the bis(phenyl)alkylenediamines correspond to the formula: 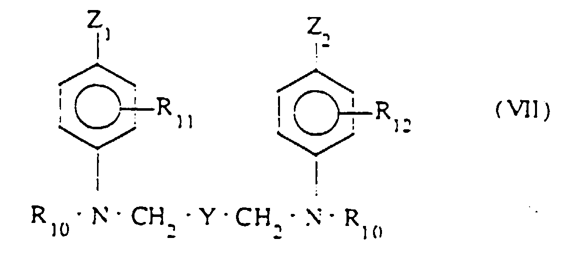 in which:
Z₁ and Z₂, which may be identical or different, represent hydroxyl groups or groups NHR₁₃, where R₁₃ denotes a hydrogen atom or a lower alkyl radical;
R₁₁ and R₁₂, which may be identical or different, represent either hydrogen atoms or halogen atoms or alternatively alkyl group [sic] ;
R₁₀ represents a hydrogen atom, an alkyl or hydroxyalkyl group or an aminoalkyl group in which the amino residue may be substituted;
Y represents a radical selected from the group consisting of the following radicals:
-(CH₂)ₙ-, (CH₂)ₘ -O-(CH₂)ₘ,
- (CH₂)_{q} - CHOH - (CH₂)_{q}, 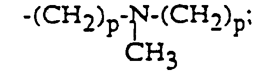
n is an integer between 0 and 8 and m, q and p are integers between 0 and 4. It being possible for this base also to take the form of its addition salts with acids.

14. Composition according to Claim 13, characterized in that the bis(phenyl)alkylenediamines are chosen from N,N'-bis(β-hydroxyethyl)-N,N'-bis(4'-aminophenyl)-1,3-diamino-2-propanol, N,N'-bis(β-hydroxyethyl)-N,N'-bis (4'-aminophenyl) ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis(ethyl)-N,N'-bis(4'-amino-3'-methylphenyl)ethylenediamine.

15. Composition according to any one of Claims 6 to 14, characterized in that the compositions contain, in addition to the coupler of formula (I) as defined above, other couplers which are known per se, chosen from meta-diphenols, meta-aminophenols, meta-phenylenediamines, meta-acylaminophenols, meta-ureidophenols, meta-carbalkoxyaminophenols, α-naphthol, indole derivatives, couplers possessing an active methylene group, such as β-keto compounds, pyrazolones.

16. Composition according to Claim 15, characterized in that the additional couplers are chosen from 2,4-dihydroxyphenoxyethanol, 2,4-dihydroxyanisole, meta-aminophenol, resorcinol monomethyl ether, resorcinol, 2-methylresorcinol, 2-methyl-5-aminophenol, 2-methyl-5-N-(β-hydroxyethyl)aminophenol, 2-methyl-5-N-(β-mesylaminoethyl)aminophenol, 2,6-dimethyl-3-aminophenol, 6-hydroxybenzomorpholine, 2,4-diaminoanisole, 2,4-diaminophenoxyethanol, 6-aminobenzomorpholine, [2-N-(β-hydroxyethyl)amino-4-amino]phenoxyethanol [sic], 2-amino-4-N-(β-hydroxyethyl) aminoanisole, (2,4-diamino)phenyl [sic] β,γ-dihydroxypropyl ether, 2,4-diaminophenoxyethylamine, 1,3-dimethoxy-2,4-diaminobenzene, 1,3,5-trimethoxy-2,4-diaminobenzene, 1-amino-3,4-methylenedioxybenzene, 1-hydroxy-3,4-methylenedioxybenzene, 2-chloro-6-methyl-3-aminophenol, 2-methyl-3-aminophenol, 2-chlororesorcinol, 6-methoxy-3-hydroxyethylaminoaniline, 1-ethoxy-2-bis-(β-hydroxyethyl)amino-4-aminobenzene, 3-diethylaminophenol, 1,3-dihydroxy-2-methylbenzene, 1-hydroxy-2,4-dichloro-3-aminobenzene, 4,6-hydroxyethoxy-1,3-diaminobenzene, 4-methyl-6-ethoxy-1,3-diaminobenzene, 4-chloro-6-methyl-3-aminophenol, 6-chloro-3-trifluoroethylaminophenol, and their salts.

17. Composition according to any one of Claims 6 to 16, characterized in that the dyeing composition contains, in addition, direct dyes chosen from azo, anthraquinone dyes or nitro derivatives of the benzene series.

18. Composition according to any one of Claims 6 to 17, characterized in that the para and/or ortho oxidation dye precursors as well as the couplers are present in the dyeing compositions in proportions of between 0.3 and 7% by weight relative to the total weight of the composition.

19. Composition according to any one of Claims 6 to 18, characterized in that the compound of formula (I) is present in proportions of between 0.05 and 3.5% by weight relative to the total weight of the composition.

20. Composition according to any one of Claims 6 to 19, characterized in that the appropriate medium for the dyeing is aqueous, and in that its pH is between 4 and 11.

21. Process for dyeing keratinous fibres, especially hair, characterized in that the dyeing is performed using a composition possessing a pH below 8 which is obtained after mixing the dyeing composition defined in any one of Claims 6 to 20 with an oxidizing solution used in sufficient amounts to be able to develop a coloration.

22. Process according to Claim 21, characterized in that the pH of the composition applied to the keratinous fibres, especially hair, is between 3.5 and 7.

23. Compound, characterized in that it corresponds to the formula: 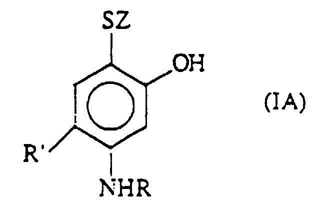 in which Z represents a C₁-C₁₈ alkyl radical, an aralkyl radical in which the alkyl radical contains 1 to 6 carbon atoms, a C₁-C₆ monohydroxyalkyl radical, a C₂-C₆ polyhydroxyalkyl radical, an aminoalkyl radical of formula: 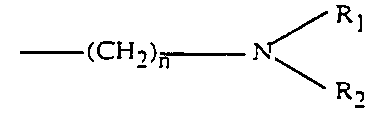 in which n is an integer between 1 and 6 inclusive, R₁ and R₂, which may be identical or different, represent a hydrogen atom or a C₁-C₄-alkyl, C₁-C₄ hydroxyalkyl, C₂-C₆ acyl radical;
R represents a hydrogen atom, a C₁-C₆ alkyl radical, a C₁-C₆ monohydroxyalkyl, C₂-C₆ polyhydroxyalkyl, monocarbamyl (C₁-C₆ alkyl), dicarbamyl (C₁-C₆ alkyl), C₁-C₆ aminoalkyl, C₁-C₆ acyl, C₂-C₆ carbalkoxy, carbamyl or mono(C₁-C₆ alkyl)carbamyl radical, R' represents a hydrogen atom, a C₁-C₄ alkyl, C₁-C₄ thioalkyl or C₁-C₄ alkoxy radical, with the proviso that, when R and R' denote a hydrogen atom, Z cannot denote butyl, β,γ-dihydroxypropyl and diethylaminoethyl radicals, as well as the corresponding salts with an acid.

24. Process for preparing the compounds according to Claim 23, characterized in that 3,4-methylenedioxy-1-nitrobenzene, optionally substituted at position 6 with alkyl, thioalkyl or alkoxy, is reacted in the presence of a base with a thiol of formula (II) :
Z-SH (II)
in which Z represents a C₁-C₁₈ alkyl group, an aralkyl group in which the alkyl group is a C₁-C₆ radical, a monohydroxylated C₁-C₆ alkyl group or a polyhydroxylated C₂-C₆ alkyl group, a group of formula: 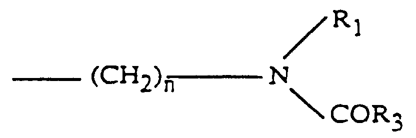 where R₁ and n have the meanings stated above and where R₃ represents a hydrogen atom or an alkyl radical having 1 to 3 carbon atoms; in a second step, the NO₂ group of the compound of formula (III): 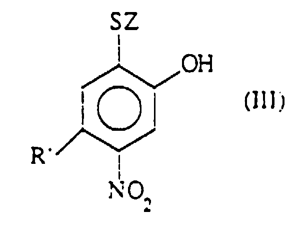 obtained above, is reduced to prepare a compound corresponding to the formula (IV): 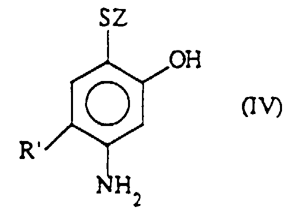 in which Z has the meaning stated above: and in that, if necessary, in a third step, conversion of the compound (IV) is carried out, either by acid hydrolysis when Z comprises an acylated amine group, or by substitution of the extranuclear amine when Z contains an amine group, or by monosubstutution of the aromatic amine, to obtain the compounds corresponding to the formula (IA).
